# EUROPEAN PATENT APPLICATION

(11) **EP 2 368 980 A2**
(43) Date of publication of application: **28.09.2011**
(21) Application number: 10012257.1
(22) Date of filing: 03.10.2006
(51) Int. Cl.: C12N 9/22

(54) **I-crel homing endonuclease variants having novel claevage specificity and use thereof**

(30) Priority: 25.10.2005 WO PCT/IB2005/003564
(62) Divisional of application: 06842325.0
(71) Applicant: Cellectis, 93235 Romainville Cedex (FR)
(72) Inventor: Paques, Frédéric, 92340 Bourg-La-Reine (FR)
(74) Representative: Noel, Chantal Odile

(57) **Abstract**

A method for engineering I-*Cre*I homing endonuclease variants able to cleave mutant I*-Cre*I sites having variation in positions ± 8 to ±10. A I-*Cre*I homing endonuclease variant obtainable by said method, a vector encoding said variant, a cell, an animal or a plant modified by said vector. Use of said I-*Cre*I endonuclease variant and derived products for genetic engineering, genome therapy and antiviral therapy.

SEQUENCE LISTING

<110>  CELLECTIS
       PAQUES, Frederic

<120>  I-CreI homing endonuclease variants having novel cleavage
       specificity and use thereof

<130>  1546PCT8

<160>  75

<170>  PatentIn version 3.3

<210>  1
<211>  24
<212>  DNA
<213>  ARTIFICIAL SEQUENCE

<220>
<223>  OLIGONUCLEOTIDE

<400>  1
tcaaaacgtc gtgagacagt ttgg                                              24


<210>  2
<211>  24
<212>  DNA
<213>  ARTIFICIAL SEQUENCE

<220>
<223>  OLIGONUCLEOTIDE

<400>  2
ccaaactgtc tcgagacagt ttgg                                              24


<210>  3
<211>  24
<212>  DNA
<213>  ARTIFICIAL SEQUENCE

<220>
<223>  OLIGONUCLEOTIDE

<400>  3
tcaaaacgtc gtacgacgtt ttga                                              24


<210>  4
<211>  24
<212>  DNA
<213>  ARTIFICIAL SEQUENCE

<220>
<223>  OLIGONUCLEOTIDE

<400>  4
tcgggacgtc gtacgacgtc ccga                                              24


<210>  5
<211>  24
<212>  DNA
<213>  ARTIFICIAL SEQUENCE

<220>

<223>  OLIGONUCLEOTIDE

<400>  5
tcggaacgtc gtacgacgtt ccga                                              24


<210>  6
<211>  24
<212>  DNA
<213>  ARTIFICIAL SEQUENCE

<220>
<223>  OLIGONUCLEOTIDE

<400>  6
tcggtacgtc gtacgacgta ccga                                              24


<210>  7
<211>  24
<212>  DNA
<213>  ARTIFICAIL SEQUENCE

<400>  7
tcggcacgtc gtacgacgtg ccga                                              24


<210>  8
<211>  24
<212>  DNA
<213>  ARTIFICIAL SEQUENCE

<220>
<223>  OLIGONUCLEOTIDE

<400>  8
tcgagacgtc gtacgacgtc tcga                                              24


<210>  9
<211>  24
<212>  DNA
<213>  ARTIFICIAL SEQUENCE

<220>
<223>  OLIGONUCLEOTIDE

<400>  9
tcgaaacgtc gtacgacgtt tcga                                              24


<210>  10
<211>  24
<212>  DNA
<213>  artificial sequence

<220>
<223>  oligonucleotide

<400>  10
tcgatacgtc gtacgacgta tcga                                              24


<210>  11
<211>  24
<212>  DNA
<213>  artificial sequence

<220>
<223>  oligonucleotide

<400>  11
tcgacacgtc gtacgacgtg tcga                                              24


<210>  12
<211>  24
<212>  DNA
<213>  artificial sequence

<220>
<223>  oligonucleotide

<400>  12
tcgtgacgtc gtacgacgtc acga                                              24


<210>  13
<211>  24
<212>  DNA
<213>  artificial sequence

<220>
<223>  oligonucleotide

<400>  13
tcgtaacgtc gtacgacgtt acga                                              24


<210>  14
<211>  24
<212>  DNA
<213>  artificial sequence

<220>
<223>  oligonucleotide

<400>  14
tcgttacgtc gtacgacgta acga                                              24


<210>  15
<211>  24
<212>  DNA
<213>  artificial sequence

<220>
<223>  oligonucleotide

<400>  15
tcgtcacgtc gtacgacgtg acga                                              24


<210>  16
<211>  24
<212>  DNA
<213>  artificial sequence

<220>
<223>  oligonucleotide

<400>  16
tcgcgacgtc gtacgacgtc gcga                                              24


<210>  17

<211>  24
<212>  DNA
<213>  artificial sequence

<220>
<223>  oligonucleotide

<400>  17
tcgcaacgtc gtacgacgtt gcga                                              24


<210>  18
<211>  24
<212>  DNA
<213>  artificial sequence

<220>
<223>  oligonucleotide

<400>  18
tcgctacgtc gtacgacgta gcga                                              24


<210>  19
<211>  24
<212>  DNA
<213>  artificial sequence

<220>
<223>  oligonucleotide

<400>  19
tcgccacgtc gtacgacgtg gcga                                              24


<210>  20
<211>  24
<212>  DNA
<213>  artificial sequence

<220>
<223>  oligonucleotide

<400>  20
tcaggacgtc gtacgacgtc ctga                                              24


<210>  21
<211>  24
<212>  DNA
<213>  artificial sequence

<220>
<223>  oligonucleotide

<400>  21
tcagaacgtc gtacgacgtt ctga                                              24


<210>  22
<211>  24
<212>  DNA
<213>  artificial sequence

<220>
<223>  oligonucleotide

<400>  22

tcagtacgtc gtacgacgta ctga                                          24

<210>  23
<211>  24
<212>  DNA
<213>  artificial sequence

<220>
<223>  oligonucleotide

<400>  23
tcagcacgtc gtacgacgtg ctga                                          24

<210>  24
<211>  24
<212>  DNA
<213>  artificial sequence

<220>
<223>  oligonucleotide

<400>  24
tcaagacgtc gtacgacgtc ttga                                          24

<210>  25
<211>  24
<212>  DNA
<213>  artificial sequence

<220>
<223>  oligonucleotide

<400>  25
tcaaaacgtc gtacgacgtt ttga                                          24

<210>  26
<211>  24
<212>  DNA
<213>  artificial sequence

<220>
<223>  oligonucleotide

<400>  26
tcaatacgtc gtacgacgta ttga                                          24

<210>  27
<211>  24
<212>  DNA
<213>  artificial sequence

<220>
<223>  oligonucleotide

<400>  27
tcaacacgtc gtacgacgtg ttga                                          24

<210>  28
<211>  24
<212>  DNA
<213>  artificial sequence

<220>
<223>  oligonucleotide

<400>  28
tcaacacgtc gtacgacgtg ttga                                              24


<210>  29
<211>  24
<212>  DNA
<213>  artificial sequence

<220>
<223>  oligonucleotide

<400>  29
tcataacgtc gtacgacgtt atga                                              24


<210>  30
<211>  24
<212>  DNA
<213>  artificial sequence

<220>
<223>  oligonucleotide

<400>  30
tcattacgtc gtacgacgta atga                                              24


<210>  31
<211>  24
<212>  DNA
<213>  artificial sequence

<220>
<223>  oligonucleotide

<400>  31
tcatcacgtc gtacgacgtg atga                                              24


<210>  32
<211>  24
<212>  DNA
<213>  artificial sequence

<220>
<223>  oligonucleotide

<400>  32
tcatcacgtc gtacgacgtg atga                                              24


<210>  33
<211>  24
<212>  DNA
<213>  artificial sequence

<220>
<223>  oligonucleotide

<400>  33
tcatcacgtc gtacgacgtg atga                                              24


<210>  34

<211>  24
<212>  DNA
<213>  artificial sequence

<220>
<223>  oligonucleotide

<400>  34
tcactacgtc gtacgacgta gtga                                              24


<210>  35
<211>  24
<212>  DNA
<213>  artificial sequence

<220>
<223>  oligonucleotide

<400>  35
tcaccacgtc gtacgacgtg gtga                                              24


<210>  36
<211>  24
<212>  DNA
<213>  artificial sequence

<220>
<223>  oligonucleotide

<400>  36
tctggacgtc gtacgacgtc caga                                              24


<210>  37
<211>  24
<212>  DNA
<213>  artificial sequence

<220>
<223>  oligonucleotide

<400>  37
tctgaacgtc gtacgacgtt caga                                              24


<210>  38
<211>  24
<212>  DNA
<213>  artificial sequence

<220>
<223>  oligonucleotide

<400>  38
tctgtacgtc gtacgacgta caga                                              24


<210>  39
<211>  24
<212>  DNA
<213>  artificial sequence

<220>
<223>  oligonucleotide

<400>  39

tctgcacgtc gtacgacgtg caga                                          24

<210>  40
<211>  24
<212>  DNA
<213>  artificial sequence

<220>
<223>  oligonucleotide

<400>  40
tctgcacgtc gtacgacgtg caga                                          24

<210>  41
<211>  24
<212>  DNA
<213>  artificial sequence

<220>
<223>  oigonucleotide

<400>  41
tctaaacgtc gtacgacgtt taga                                          24

<210>  42
<211>  24
<212>  DNA
<213>  artificial sequence

<220>
<223>  oligonucleotide

<400>  42
tctatacgtc gtacgacgta taga                                          24

<210>  43
<211>  24
<212>  DNA
<213>  artificial sequence

<220>
<223>  oligonucleotide

<400>  43
tctacacgtc gtacgacgtg taga                                          24

<210>  44
<211>  24
<212>  DNA
<213>  artificial sequence

<220>
<223>  oligonucleotide

<400>  44
tcttgacgtc gtacgacgtc aaga                                          24

<210>  45
<211>  24
<212>  DNA
<213>  artificial sequence

<220>
<223>  oligonucleotide

<400>  45
tcttaacgtc gtacgacgtt aaga                                              24


<210>  46
<211>  24
<212>  DNA
<213>  artificial sequence

<220>
<223>  oligonucleotide

<400>  46
tctttacgtc gtacgacgta aaga                                              24


<210>  47
<211>  24
<212>  DNA
<213>  artificial sequence

<220>
<223>  oligoonucleotide

<400>  47
tcttcacgtc gtacgacgtg aaga                                              24


<210>  48
<211>  24
<212>  DNA
<213>  artificial sequence

<220>
<223>  oligonucleotide

<400>  48
tctcgacgtc gtacgacgtc gaga                                              24


<210>  49
<211>  24
<212>  DNA
<213>  artificial sequence

<220>
<223>  oligonucleotide

<400>  49
tctcaacgtc gtacgacgtt gaga                                              24


<210>  50
<211>  24
<212>  DNA
<213>  artificial sequence

<220>
<223>  oligonucleotide

<400>  50
tctctacgtc gtacgacgta gaga                                              24


<210>  51

<211>  24
<212>  DNA
<213>  artificial sequence

<220>
<223>  oligonucleotide

<400>  51
tctccacgtc gtacgacgtg gaga                                              24


<210>  52
<211>  24
<212>  DNA
<213>  artificial sequence

<220>
<223>  oligonucleotide

<400>  52
tccggacgtc gtacgacgtc cgga                                              24


<210>  53
<211>  24
<212>  DNA
<213>  artificial sequence

<220>
<223>  oligonucleotide

<400>  53
tccgaacgtc gtacgacgtt cgga                                              24


<210>  54
<211>  24
<212>  DNA
<213>  artificial sequence

<220>
<223>  oligonucleotide

<400>  54
tccgtacgtc gtacgacgta cgga                                              24


<210>  55
<211>  24
<212>  DNA
<213>  artificial sequence

<220>
<223>  oligonucleotide

<400>  55
tccgcacgtc gtacgacgtg cgga                                              24


<210>  56
<211>  24
<212>  DNA
<213>  artificial sequence

<220>
<223>  oligonucleotide

<400>  56

tccagacgtc gtacgacgtc tgga 24

<210> 57
<211> 24
<212> DNA
<213> artificial sequence

<220>
<223> oligonucleotide

<400> 57
tccaaacgtc gtacgacgtt tgga 24

<210> 58
<211> 24
<212> DNA
<213> artificial sequence

<220>
<223> oligonucleotide

<400> 58
tccatacgtc gtacgacgta tgga 24

<210> 59
<211> 24
<212> DNA
<213> artificial sequence

<220>
<223> oligonucleotide

<400> 59
tccacacgtc gtacgacgtg tgga 24

<210> 60
<211> 24
<212> DNA
<213> artificial sequence

<220>
<223> oligonucleotide

<400> 60
tcctgacgtc gtacgacgtc agga 24

<210> 61
<211> 24
<212> DNA
<213> artificial sequence

<220>
<223> oligonucleotide

<400> 61
tcctaacgtc gtacgacgtt agga 24

<210> 62
<211> 24
<212> DNA
<213> artificial sequence

<220>
<223>  oligonucleotide

<400>  62
tccttacgtc gtacgacgta agga                                              24


<210>  63
<211>  24
<212>  DNA
<213>  artificial sequence

<220>
<223>  oligonucleotide

<400>  63
tcctcacgtc gtacgacgtg agga                                              24


<210>  64
<211>  24
<212>  DNA
<213>  artificial sequence

<220>
<223>  oligonucleotide

<400>  64
tcccgacgtc gtacgacgtc ggga                                              24


<210>  65
<211>  24
<212>  DNA
<213>  artificial sequence

<220>
<223>  oligonucleotide

<400>  65
tcccaacgtc gtacgacgtt ggga                                              24


<210>  66
<211>  24
<212>  DNA
<213>  artificial sequence

<220>
<223>  artificial sequence

<400>  66
tccctacgtc gtacgacgta ggga                                              24


<210>  67
<211>  24
<212>  DNA
<213>  artificial sequence

<220>
<223>  oligonucleotide

<400>  67
tccccacgtc gtacgacgtg ggga                                              24


<210>  68

<211>  501
<212>  DNA
<213>  ARTIFICIAL SEQUENCE

<220>
<223>  I-CreI N75 scaffold

<400>  68
atggccaata ccaaatataa caaagagttc ctgctgtacc tggccggctt tgtggacggt      60

gacggtagca tcatcgctca gattaaacca aaccagtctt ataagtttaa acatcagcta     120

agcttgacct ttcaggtgac tcaaaagacc cagcgccgtt ggtttctgga caaactagtg     180

gatgaaattg gcgttggtta cgtacgtgat cgcggatccg tttccaacta catcttaagc     240

gaaatcaagc cgctgcacaa cttcctgact caactgcagc cgtttctgaa actgaaacag     300

aaacaggcaa acctggttct gaaaattatc gaacagctgc cgtctgcaaa agaatccccg     360

gacaaattcc tggaagtttg tacctgggtg gatcagattg cagctctgaa cgattctaag     420

acgcgtaaaa ccacttctga aaccgttcgt gctgtgctgg acagcctgag cgagaagaag     480

aaatcctccc cggcggccga c                                               501


<210>  69
<211>  68
<212>  DNA
<213>  ARTIFICIAL SEQUENCE

<220>
<223>  OLIGONUCLEOTIDE

<400>  69
ctgaaaggtc aagcttagmb batgtttaaa cttmbbagac tgmbbtggtt taatctgagc      60

gatgatgc                                                               68


<210>  70
<211>  68
<212>  DNA
<213>  ARTIFICIAL SEQUENCE

<220>
<223>  OLIGONUCLEOTIDE

<400>  70
ctgaaaggtc aagcttagmb batgtttaaa cttataagac tgmbbtggmb baatctgagc      60

gatgatgc                                                               68


<210>  71
<211>  18
<212>  DNA
<213>  ARTIFCIAL SEQUENCE

<400>  71
ctaagcttga cctttcag                                                    18


<210>  72
<211>  49
<212>  DNA
<213>  ARTIFICIAL SEQUENCE

<220>
<223>  OLIGONUCLEOTIDE


<220>
<221>  misc_feature
<222>  (15)..(17)
<223>  n is a, c, g, or t

<220>
<221>  misc_feature
<222>  (32)..(34)
<223>  n is a, c, g, or t

<400>  72
ggcatacaag tttcnnnacg tcgtacgacg tnnngacaat cgtctgtca                49


<210>  73
<211>  26
<212>  DNA
<213>  ARTIFICIAL SEQUENCE

<220>
<223>  OLIGONUCLEOTIDE

<400>  73
gcaactttag tgctgacaca tacagg                                         26


<210>  74
<211>  24
<212>  DNA
<213>  ARTIFICIAL SEQUENCE

<220>
<223>  artificial sequence

<400>  74
acaaccttga ttgcagactt gacc                                           24


<210>  75
<211>  21
<212>  DNA
<213>  ARTIFICIAL SEQUENCE

<220>
<223>  OLIGONUCLEOTIDE


<220>
<221>  misc_feature
<222>  (2)..(4)
<223>  n is a, c, g, or t

<220>
<221>  misc_feature
<222>  (6)..(8)
<223>  n is a, c, g, or t

<220>
<221>  misc_feature
<222>  (13)..(15)
<223>  n is a, c, g, or t

<220>

<221>  misc_feature
<222>  (18)..(20)
<223>  n is a, c, g, or t

<400>  75
cnnnmnnnky rmnnnrknnn g                                                21

## Description

The invention relates to a method for engineering *I-CreI* homing endonuclease variants able to cleave mutant *I-CreI* sites having variation in positions ± 8 to ±10. The invention relates also to an *I-Crel* homing endonuclease variant obtainable by said method, to a vector encoding said variant, to a cell, an animal or a plant modified by said vector and to the use of said I-CreI endonuclease variant and derived products for genetic engineering, genome therapy and antiviral therapy.

Meganucleases are by definition sequence-specific endonucleases with large (>14 bp) cleavage sites that can deliver DNA double-strand breaks (DSBs) at specific loci in living cells (Thierry and Dujon, Nucleic Acids Res., 1992, 20, 5625-5631). Meganucleases have been used to stimulate homologous recombination in the vicinity of their target sequences in cultured cells and plants (Rouet et al., Mol. Cell. Biol., 1994, 14, 8096-106; Choulika et al., Mol. Cell. Biol., 1995, 15, 1968-73; Donoho et al., Mol. Cell. Biol, 1998, 18, 4070-8; Elliott et al., Mol. Cell. Biol., 1998, 18, 93-101; Sargent et al., Mol. Cell. Biol., 1997, 17, 267-77; Puchta et al., Proc. Natl. Acad. Sci. USA, 1996, 93, 5055-60; Chiurazzi et al., Plant Cell, 1996, 8, 2057-2066), making meganuclease-induced recombination an efficient and robust method for genome engineering. The use of meganuclease-induced recombination has long been limited by the repertoire of natural meganucleases, and the major limitation of the current technology is the requirement for the prior introduction of a meganuclease cleavage site in the locus of interest.

Thus, the making of artificial meganucleases with tailored substrate specificities is under intense investigation. Such proteins could be used to cleave genuine chromosomal sequences and open new perspectives for genome engineering in wide range of applications. For example, meganucleases could be used to induce the correction of mutations linked with monogenic inherited diseases, and bypass the risk due to the randomly inserted transgenes used in current gene therapy approaches (Hacein-Bey-Abina et al., Science, 2003, 302, 415-419).

Recently, Zinc-Finger DNA binding domains of Cys2-His2 type Zinc-Finger Proteins (ZFP) could be fused with the catalytic domain of the *Fok*I endonuclease, to induce recombination in various cell types, including human lymphoid cells (Smith et al., Nucleic Acids Res, 1999, 27, 674-81; Pabo et al., Annu. Rev. Biochem, 2001, 70, 313-40; Porteus and Baltimore, Science, 2003, 300, 763; Urnov et al., Nature, 2005, 435, 646-651; Bibikova et al., Science, 2003, 300, 764). The binding specificity of ZFPs is relatively easy to manipulate, and a repertoire of novel artificial ZFPs, able to bind many (g/a)nn(g/a)nn(g/a)nn sequences is now available (Pabo et al., precited; Segal and Barbas, Curr. Opin. Biotechnol., 2001, 12, 632-7; Isalan et al., Nat. Biotechnol., 2001, 19, 656-60). However, preserving a very narrow substrate specificity is one of the major issues for genome engineering applications, and presently it is unclear whether ZFPs would fulfill the very strict requirements for therapeutic applications. Furthermore, these fusion proteins have demonstrated high toxicity in cells (Porteus and Baltimore, precited; Bibikova et al., Genetics, 2002, 161, 1169-1175)), probably due to a low level of specificity.

In nature, meganucleases are essentially represented by homing endonucleases (HEs), a family of endonucleases encoded by mobile genetic elements, whose function is to initiate DNA double-strand break (DSB)-induced recombination events in a process referred to as homing (Chevalier and Stoddard, Nucleic Acids Res., 2001, 29, 3757-74; Kostriken et al., Cell; 1983, 35, 167-74; Jacquier and Dujon, Cell, 1985, 41, 383-94). Several hundreds of HES have been identified in bacteria, eukaryotes, and archea (Chevalier and Stoddard, precited); however the probability of finding a HE cleavage site in a chosen gene is very low.

Given their biological function and their exceptional cleavage properties in terms of efficacy and specificity, HEs provide ideal scaffolds to derive novel endonucleases for genome engineering. Data have been accumulated over the last decade, characterizing the LAGLIDADG family, the largest of the four HE families (Chevalier and Stoddard, precited). LAGLIDADG refers to the only sequence actually conserved throughout the family and is found in one or (more often) two copies in the protein. Proteins with a single motif, such as I-*CreI*, form homodimers and cleave palindromic or pseudo-palindromic DNA sequences, whereas the larger, double motif proteins, such as *I-SceI* are monomers and cleave non palindromic targets. Seven different LAGLIDADG proteins have been crystallized, and they exhibit a very striking conservation of the core structure, that contrasts with the lack of similarity at the primary sequence level (Jurica et al., Mol. Cell., 1998, 2, 469-76; Chevalier et al., Nat. Struct. Biol., 2001, 8, 312-6 ; Chevalier et al. J. Mol. Biol., 2003, 329, 253-69; Moure et al., J. Mol. Biol, 2003, 334, 685-95; Moure et al., Nat. Struct. Biol., 2002, 9, 764-70; Ichiyanagi et al., J. Mol. Biol., 2000, 300, 889-901; Duan et al., Cell, 1997, 89, 555-64; Bolduc et al., Genes Dev., 2003, 17, 2875-88; Silva et al., J. Mol. Biol., 1999, 286, 1123-36). In this core structure, two characteristic αββαββα folds, also called LAGLIDADG homing endonuclease core domains, contributed by two monomers, or by two domains in double LAGLIDAG proteins, are facing each other with a two-fold symmetry. DNA binding depends on the four β strands from each domain, folded into an antiparallel (β-sheet, and forming a saddle on the DNA helix major groove. Analysis of I-*CreI* structure bound to its natural target shows that in each monomer, eight residues (Y33, Q38, N30, K28, Q26, Q44, R68 and R70) establish direct interaction with seven bases at positions ± 3, 4, 5, 6, 7, 9 and 10 (Jurica *et al.,* 1998, precited). In addition, some residues establish water-mediated contact with several bases; for example S40, K28 and N30 with the base pair at position +8 and -8 (Chevalier *et al.,* 2003, precited). The catalytic core is central, with a contribution of both symmetric monomers/domains. In addition to this core structure, other domains can be found: for example, PI-*Sce*I, an intein, has a protein splicing domain, and an additional DNA-binding domain (Moure *et al.,* 2002, precited; Grindl et al., Nucleic Acids Res., 1998, 26, 1857-62).

Two approaches for deriving novel meganucleases from homing endonucleases, are under investigation:

### - hybrid or chimeric single-chain proteins

New meganucleases could be obtained by swapping LAGLIDADG homing endonuclease core domains of different monomers (Epinat et al., Nucleic Acids Res., 2003, 31, 2952-62; Chevalier et al., Mol. Cell., 2002, 10, 895-905; Steuer et al., Chembiochem., 2004, 5, 206-13; International PCT Applications WO 03/078619 and WO 2004/031346). These single-chain chimeric meganucleases wherein the two LAGLIDADG homing endonuclease core domains from different meganucleases are linked by a spacer, are able to cleave the hybrid target corresponding to the fusion of the two half parent DNA target sequences. These results mean that the two DNA binding domain of an I*-CreI* dimer behave independently; each DNA binding domain binds a different half of the DNA target site. The construction of chimeric and single chain artificial HEs has suggested that a combinatorial approach could be used to obtain novel meganucleases cleaving novel (non-palindromic) target sequences: different monomers or core domains could be fused in a single protein, to achieve novel specificities.

However, this approach does not enrich considerably the number of DNA sequences that can be targeted with homing endonucleases since the novel targets which are generated result from the combination of two different DNA target half-sites.

### - protein variants

Altering the substrate specificity of DNA binding proteins by mutagenesis and screening/selection has often proven to be difficult (Lanio et al., Protein Eng., 2000, 13, 275-281; Voziyanov et al., J. Mol. Biol., 2003, 326, 65-76; Santoro et al., P.N.A.S., 2002, 99, 4185-4190; Buchholz and Stewart, Nat. Biotechnol., 2001, 19, 1047-1052), and more particularly, engineering HEs DNA binding domain has long been considered a daunting task (Ashworth et al., Nature 2006, 441, 656-659; Gimble et al., J. Mol. Biol., 2003, 334, 993-1008 ; Arnould et al., J. Mol. Biol., 2006, 355, 443-458; Doyon et al., J. Am. Chem. Soc., 2006, 128, 2477-2484; Steuer *et al.,* precited; Seligman et al., Nucleic Acids Res., 2002, 30, 3870-3879).

Analysis of the I*-CreI* / DNA crystal structure indicates that 9 amino acids make direct contacts with the homing site (Chevalier *et al.,* 2003; Jurica et al., precited) which randomization would result in 20⁹ combinations, a number beyond any screening capacity today.

Therefore, several laboratories have relied on a semi-rational approach (Chica et al., Curr. Opin. Biotechnol., 2005, 16, 378-384) to limit the diversity of the mutant libraries to be handled: a small set of relevant residues is chosen according to structural data. Nevertheless, this was still not sufficient to create redesigned endonucleases cleaving chosen sequences:
- Seligman and co-workers used a rational approach to substitute specific individual residues of the I*-Cre*I αββαββα fold (Sussman et al., J. Mol. Biol., 2004, 342, 31-41; Seligman et al., Nucleic Acids Res., 2002, precited ; Seligman et al., Genetics, 1997, 147, 1653-64). However, substantial cleavage was observed with few I*-CreI* variants (Y33C, Y33H, Y33R, Y33L, Y33S, Y33T, S32K, S32R) and only for a target modified in position ±10.
- In a similar way, Gimble *et al.* (precited) modified the additional DNA binding domain of PI*-Scel;* they obtained variant protein with altered binding specificity but no altered substrate specificity and most of the proteins maintained a lot of affinity for the wild-type target sequence.

To reach a larger number of sequences, it would be extremely valuable to be able to generate other homing endonuclease variants with novel substrate specificity, ie able to cleave DNA targets which are not cleaved by the parent homing endonuclease or the few variants which have been isolated so far.

In particular, it would be extremely valuable to generate homing endonuclease variants able to cleave novel DNA targets wherein several nucleotides of the wild-type meganuclease DNA target have been mutated simultaneously.

However, this approach is not easy since the HEs DNA binding interface is very compact and the two different ββ hairpins which are responsible for virtually all base-specific interactions are part of a single fold. Thus, the mutation of several amino acids placed in close vicinity which is required for binding a target mutated at several positions may disrupt the structure of the binding interface.

The Inventor has engineered hundreds of novel *I-CreI* variants which, altogether, target all of the 64 possible mutant *I-CreI* sites differing at positions ± 10, ± 9, and ± 8. These variants having new substrate specificity towards nucleotides ± 8, ± 9, and/or ± 10, increase the number of DNA sequences that can be targeted with meganucleases. Potential applications include genetic engineering, genome engineering, gene therapy and antiviral therapy.

Thus, the invention concerns a method for engineering a *I-CreI* homing endonuclease variant having a modified cleavage specificity, comprising at least the steps of :
(a) replacing at least one of the amino acids K28, N30, Y33, Q38 and/or S40 from the β₁β₂ hairpin of *I-Crel,* with an amino acid selected from the group consisting of A, C, D, E, G, H, K, N, P, Q, R, S, T, L, V, W, and Y
(b) selecting and/or screening the *I-CreI* variants from step (a) which are able to cleave a DNA target sequence consisting of a mutant *I-CreI* site wherein at least the aa nucleotide doublet in positions -9 to -8 and/or the tt nucleotide doublet in positions +8 to +9 has been replaced with a different nucleotide doublet.

### Definitions

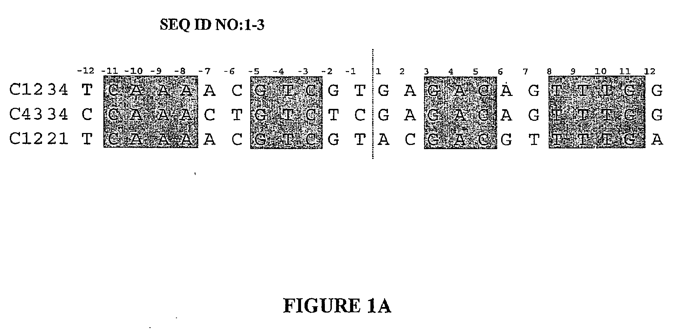

- Amino acid residues in a polypeptide sequence are designated herein according to the one-letter code, in which, for example, K means Lys or Lysine residue, N means Asn or Asparagine residue and Y means Tyr or Tyrosine residue.
- Nucleotides are designated as follows: one-letter code is used for designating the base of a nucleoside: a is adenine, t is thymine, c is cytosine, and g is guanine. For the degenerated nucleotides, r represents g or a (purine nucleotides), k represents g or t, s represents g or c, w represents a or t, m represents a or c, y represents t or c (pyrimidine nucleotides), d represents g, a or t, v represents g, a or c, b represents g, t or c, h represents a, t or c, and n represents g, a, t or c.
- by *"I-CreI"* is intended the wild-type *I-CreI* having the sequence SWISSPROT P05725 or pdb accession code 1g9y.
- by *"I-CreI* variant" or "variant" is intended a protein obtained by replacement of at least one amino acid *of I-CreI* with a different amino acid.
- by "functional I-*Cre*I variant" is intended a I-Crel variant which is able to cleave a DNA target, preferably a DNA target which is not cleaved by *I-Crel.* For example, such variants have amino acid variation at positions contacting the DNA target sequence or interacting directly or indirectly with said DNA target.
- by "homing endonuclease domain" or "domain" is intended the region which interacts with one half of the DNA target of a homing endonuclease and is able to associate with the other domain of the same homing endonuclease which interacts with the other half of the DNA target to form a functional endonuclease able to cleave said DNA target.
- by "core domain" is intended the "LAGLIDADG homing endonuclease core domain" which is the characteristic α₁β₁β₂α₂β₃β₄α₃ fold of the homing endonucleases of the LAGLIDADG family, corresponding to a sequence of about one hundred amino acid residues. Said domain comprises four beta-strands (β₁, β₂, β₃, β₄) folded in an antiparallel beta-sheet which interacts with one half of the DNA target. For example, in the case of the dimeric homing endonuclease *I-Cre*I (163 amino acids), the LAGLIDADG homing endonuclease core domain corresponds to the residues 6 to 94. In the case of monomeric homing endonuclease, two such domains are found in the sequence of the endonuclease; for example in I*-Dmo*I (194 amino acids), the first domain (residues 7 to 99) and the second domain (residues 104 to 194) are separated by a short linker (residues 100 to 103).
- by *"I-Crel* site" is intended a 22 to 24 bp double-stranded DNA sequence which is cleaved by I*-Cre*I*. I-CreI* sites include the wild-type (natural) non-palindromic *I-CreI* homing site and the derived palindromic sequences which are presented in figure 1A, such as the sequence 5'- t-₁₂c-₁₁a-₁₀a-₉a-₈a-₇c-₆g-₅t-₄c-₃g-₂t-₁a+₁c+₂g+₃a+₄c+₅g+₆t+₇t+₈t+₉t+₁₀g+₁₁a+₁₂ also called C1221 (SEQ ID NO :3).
- by "DNA target". "DNA target sequence", "target sequence" , "target" , "recognition site", "recognition sequence", "homing recognition site", "homing site", "cleavage site" is intended a 22 to 24 bp double-stranded palindromic, partially palindromic (pseudo-palindromic) or non-palindromic polynucleotide sequence that is recognized and cleaved by a meganuclease, for example a LAGLIDADG homing endonuclease such as *I-CreI,* or a variant or a single-chain chimeric meganuclease derived from said meganuclease. These terms refer to a distinct DNA location, preferably a genomic location, at which a double-stranded break (cleavage) is to be induced by the endonuclease. The DNA target is defined by the 5' to 3' sequence of one strand of the double-stranded polynucleotide.
- by "DNA target half-site" is intended the portion of the DNA target which is bound by each LAGLIDADG homing endonuclease core domain.
- by "chimeric DNA target" or "hybrid DNA target" is intended the fusion of a different half of each parent meganuclease DNA target sequence.
- by "homing endonuclease variant with novel specificity" is intended a variant having a pattern of cleaved targets different from that of the parent homing endonuclease. The terms "novel specificity", "modified specificity", "novel cleavage specificity", "novel substrate specificity" which are equivalent and used indifferently, refer to the specificity of the variant towards the nucleotides of the DNA target sequence.
- by "vector" is intended a nucleic acid molecule capable of transporting another nucleic acid to which it has been linked.
- by "homologous" is intended a sequence with enough identity to another one to lead to a homologous recombination between sequences, more particularly having at least 95 % identity, preferably 97 % identity and more preferably 99 %.
- "Identity" refers to sequence identity between two nucleic acid molecules or polypeptides. Identity can be determined by comparing a position in each sequence which may be aligned for purposes of comparison. When a position in the compared sequence is occupied by the same base, then the molecules are identical at that position. A degree of similarity or identity between nucleic acid or amino acid sequences is a function of the number of identical or matching nucleotides at positions shared by the nucleic acid sequences. Various alignment algorithms and/or programs may be used to calculate the identity between two sequences, including FASTA, or BLAST which are available as a part of the GCG sequence analysis package (University of Wisconsin, Madison, Wis.), and can be used with, e.g., default settings.
- "individual" includes mammals, as well as other vertebrates (e.g., birds, fish and reptiles). The terms "mammal" and "mammalian", as used herein, refer to any vertebrate animal, including monotremes, marsupials and placental, that suckle their young and either give birth to living young (eutharian or placental mammals) or are egg-laying (metatharian or nonplacental mammals). Examples of mammalian species include humans and other primates (e.g., monkeys, chimpanzees), rodents (e.g., rats, mice, guinea pigs) and ruminants (e.g., cows, pigs, horses).
- "genetic disease" refers to any disease, partially or completely, directly or indirectly, due to an abnormality in one or several genes. Said abnormality can be a mutation, an insertion or a deletion. Said mutation can be a punctual mutation. Said abnormality can affect the coding sequence of the gene or its regulatory sequence. Said abnormality can affect the structure of the genomic sequence or the structure or stability of the encoded mRNA. Said genetic disease can be recessive or dominant. Such genetic disease could be, but are not limited to, cystic fibrosis, Huntington's chorea, familial hyperchoiesterolemia (LDL receptor defect), hepatoblastoma, Wilson's disease, congenital hepatic porphyrias, inherited disorders of hepatic metabolism, Lesch Nyhan syndrome, sickle cell anemia, thalassaemias, xeroderma pigmentosum, Fanconi's anemia, retinitis pigmentosa, ataxia telangiectasia, Bloom's syndrome, retinoblastoma, Duchenne's muscular dystrophy, and Tay-Sachs disease.

According to the method of the invention, the amino acid mutation(s) in step a) are introduced in either wild-type *I-CreI* or a functional variant thereof. Step a) may comprise the introduction of additional mutations, particularly at other positions contacting the DNA target sequence or interacting directly or indirectly with said DNA target. Functional variants comprise mutations that do not affect the protein structure. For example, the amino acid mutations in step(a) may be introduced in an I-*Crel* variant comprising one or more mutations selected from the group consisting of:
- the mutation of the isoleucine in position 24 in a valine (I24V),
- the mutation of the arginine in position 70, in a serine (R70S), and
- the mutation of the aspartic acid in position 75, in an uncharged amino acid, preferably an asparagine (D75N) or a valine (D75V).

Step a) may be performed by generating a library of variants as described in the International PCT Application WO 2004/067736.

The selection and/or screening in step (b) may be performed by using a cleavage assay *in vitro* or *in vivo,* as described in the International PCT Application WO 2004/067736.

According, to an advantageous embodiment of said method, the DNA target in step b) derives from a *I-Crel* site which is selected from C1234, C4334 and C1221 (SEQ ID NO: 1 to 3, figure 1A).

According to another advantageous embodiment of said method, the DNA target in step b) comprises a sequence having the formula:

c-₁₁n-₁₀n-₉n-₈m-₇y-₆n-₅n-₄n-₃k-₂y-₁ r+₁m+₂n+₃n+₄n+₅r+₆k+₇n+8ₙ+₉n+₁₀g+₁₁ (I),

wherein n is a, t, c, or g, m is a or c, y is c or t, k is g or t, r is a or g (SEQ ID NO: 75), providing that when n₋₉n₋₈ is as then n₊₈n₊₉ is different from tt and when n₊₈n₊₉ is tt, then n₋₉n₋₈ is different from as.

According to a preferred embodiment of said method, n₋₅n₋₄n₋₃ is gtc and/or n₊₃n₊₄n₊₅ is gac.

The DNA target in step b) may be palindromic, non-palindromic or pseudo-palindromic. Preferably, the nucleotide sequence from positions -11 to -8 and +8 to +11 and/or the nucleotide sequence from positions -5 to -3 and/or +3 to +5 are palindromic.

According to another advantageous embodiment of said method, the DNA target in step b) comprises a nucleotide doublet in positions -9 to -8, which is selected from the group consisting of: ag, at, ac, ga, gg, gt, gc, ta, tg, tt, cg, ct, or cc, and/or a nucleotide doublet in positions +8 to +9, which is the reverse complementary sequence of said nucleotide doublet in positions -9 to -8, ie ct, ta, gt, tc, cc, ac, gc, at, ca, aa, cg, ag or gg.

According to another advantageous embodiment of said method, the DNA target in step b) further comprises the replacement of the a nucleotide in position -10 and/or the t nucleotide in position +10 of the *I-CreI* site, with a different nucleotide.

Preferably, said DNA target comprises a nucleotide triplet in positions -10 to -8, which is selected from the group consisting of: aac, aag, aat, acc, acg, act, aga, agc, agg, agt, ata, atg, cag, cga, cgg, ctg, gac, gag, gat, gcc, gga, ggc, ggg, ggt, gta, gtg, gtt, tac, tag, tat, tcc, tga, tgc, tgg, tgt or ttg, and/or a nucleotide triplet in positions +8 to +10, which is the reverse complementary sequence of said nucleotide triplet in positions -10 to -8.

According to another advantageous embodiment of said method, step (b) is *performed in vivo,* under conditions where the double-strand break in the mutated DNA target sequence which is generated by said variant leads to the activation of a positive selection marker or a reporter gene, or the inactivation of a negative selection marker or a reporter gene, by recombination-mediated repair of said DNA double-strand break.

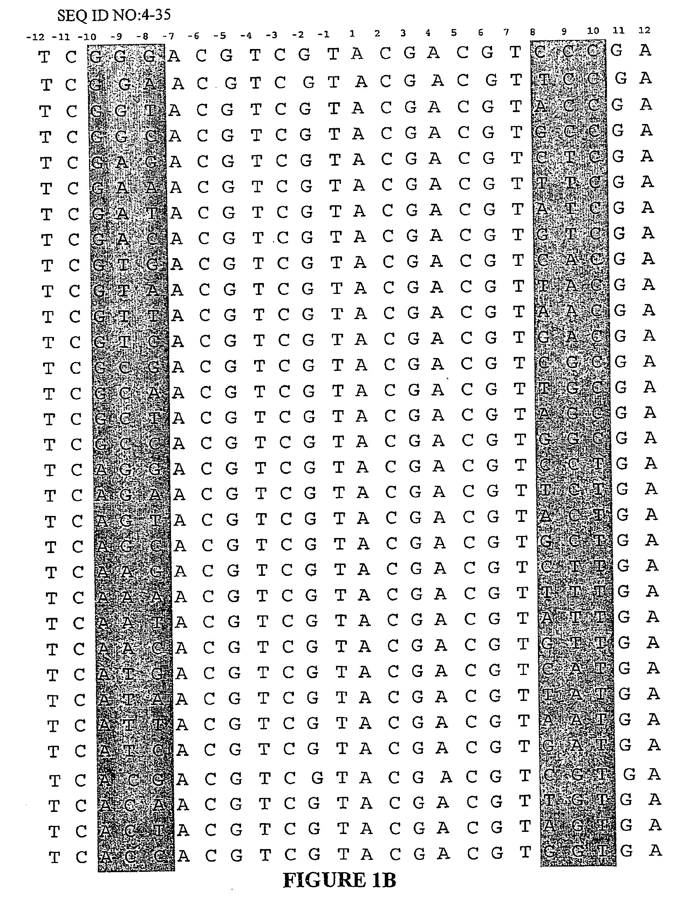

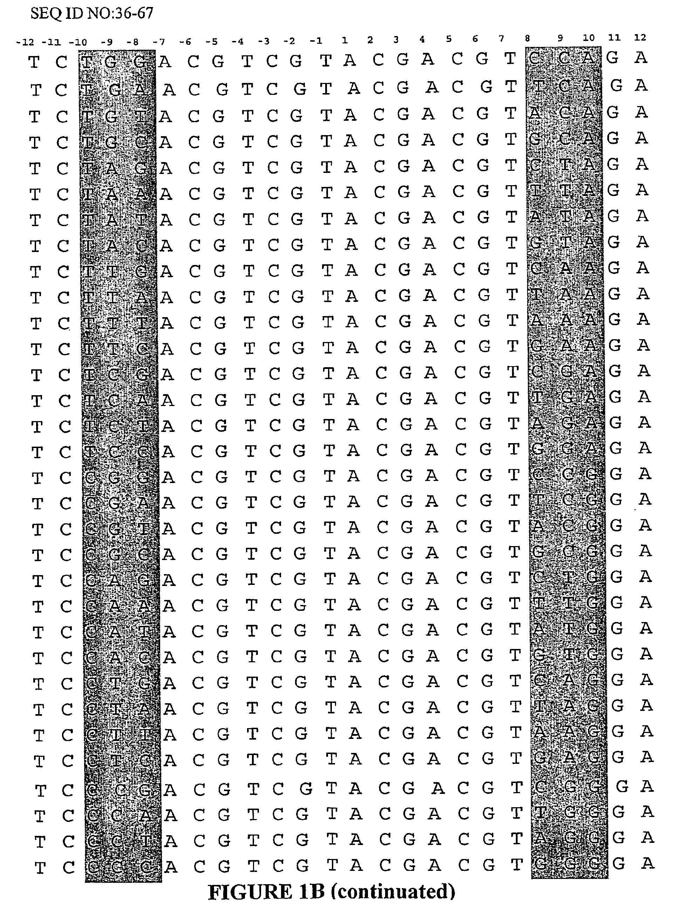

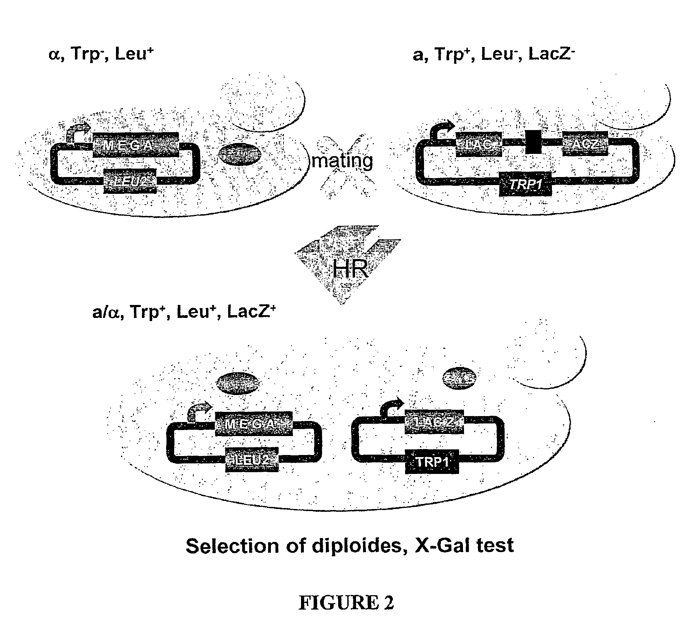

For example, the cleavage activity of the *I-CreI* variant of the invention may be measured by a direct repeat recombination assay, in yeast or mammalian cells, using a reporter vector, as described in the PCT Application WO 2004/067736. The reporter vector comprises two truncated, non-functional copies of a reporter gene (direct repeats) and a DNA target sequence within the intervening sequence, cloned in a yeast or a mammalian expression vector (Figure 2). The DNA target sequence is derived from a *I-CreI* site such as C1221, by substitution of one to three nucleotides in positions ± 8 to 10 (Figure 1B). Expression of a functional *I-CreI* variant which is able to cleave the DNA target sequence, induces homologous recombination between the direct repeats, resulting in a functional reporter gene, whose expression can be monitored by appropriate assay.

According to another advantageous embodiment of said method, it comprises a further step c₁) of expressing one variant obtained in step b), so as to allow the formation ofhomodimers.

According to another advantageous embodiment of said method, it comprises a further step c₂) of co-expressing one variant obtained in step b) and *I-CreI* or a functional variant thereof, so as to allow the formation of heterodimers. Preferably, two different variants obtained in step b) are co-expressed.

For example, host cells may be modified by one or two recombinant expression vector(s) encoding said variant(s). The cells are then cultured under conditions allowing the expression of the variant(s) and the homodimers/heterodimers which are formed are then recovered from the cell culture.

According to the method of the invention, single-chain chimeric endonucleases may be constructed by the fusion of one variant obtained in step b) with a homing endonuclease domain/monomer. Said domain/monomer may be from a wild-type homing endonuclease or a functional variant thereof.

Methods for constructing single-chain chimeric molecules derived from homing endonucleases are well-known in the art (Epinat et al., Nucleic Acids Res., 2003, 31, 2952-62; Chevalier et al., Mol. Cell., 2002, 10, 895-905; Steuer et al., Chembiochem., 2004, 5, 206-13; International PCT Applications WO 03/078619 and WO 2004/031346). Any of such methods, may be applied for constructing single-chain chimeric molecule derived from the variants as defined in the present invention.

The subject matter of the present invention is also a I*-Crel* meganuclease variant obtainable by the method as defined above, said variant being able to cleave a DNA target sequence consisting of a mutant I*-Crel* site wherein at least one of the a nucleotides in position -9 and -8, or one of the t nucleotides in position +8 and +9 has been replaced with a different nucleotide.

According to an advantageous embodiment of said I*-CreI* variant, it has an arginine (R) or a lysine (K) in position 38; the variants having R or K in position 38 are able to cleave a DNA target comprising a guanine in position -9 or a cytosine in position +9.

Said variant may be selected from the variants having amino acid residues in positions 28, 30, 33, 38 and 40 respectively, which are selected from the group consisting of: Q28/N30/Y33/K38/R40, R28/N30/K33/R38/Q40, Q28/N30/R33/R38/R40, Q28/N30/Y33/K38/K40, K28/N30/T33/R38/Q40, K28/N30/S33/R38/E40, S28/N30/Y33/R38/K40, K28/N30/S33/R38/D40, K28/N30/S33/R38/S40, Q28/N30/Y33/R38/K40, Q28/N30/K33/R38/T40, N28/N30/S33/R38/K40, N28/N30/S33/R38/R40, E28/N30/R33/R38/K40, R28/N30/T33/R38/A40, Q28/N30/Y33/R38/A40, Q28/N30/Y33/R38/S40, K28/N30/R33/K38/A40, R28/N30/A33/K38/S40, A28/N30/N33/R38/K40, Q28/N30/S33/R38/K40, K28/A30/H33/R38/S40, K28/H30/H33/R38/S40, K28/E30/S33/R38/S40, K28/N30/H33/R38/S40, K28/D30/H33/K38/S40, K28/K30/H33/R38/S40, K28/S30/H33/R38/S40, and K28/G30/V33/R38/S40.

According to another advantageous embodiment of said *I-CreI* variant, it has amino acid residues in positions 28, 30, 33, 38 and 40 respectively, which are selected from the group consisting of: Q28/N30/Y33/K38/R40, R28/N30/K33/R38/Q40, Q28/N30/R33/R38/R40, Q28/N30/Y33/K38/K40, Q28/N30/T33/Q38/K40, Q28/N30/R33/R38/K40, K28/N30/T33/Q38/R40, S28/N30/R33/S38/R40, N28/N30/Y33/Q38/R40, K28/N30/T33/R38/Q40, K28/N30/S33/R38/E40, Q28/N30/N33/Q38/K40, S28/N30/Y33/R38/K40, K28/N30/S33/R38/D40, K28/N30/R33/E38/R40, K28/N30/S33/R38/S40, R28/N30/R33/D38/R40, A28/N30/S33/Q38/R40, Q28/N30/Y33/R38/K40, Q28/N30/K33/R38/T40, R28/N30/A33/Y38/Q40, K28/N30/R33/Q38/E40, N28/N30/S33/R38/K40, N28/N30/S33/R38/R40, Q28/N30/Y33/Q38/K40, Q28/N30/Y33/Q38/R40, S28/N30/R33/Q38/R40, Q28/N30/R33/Q38/K40, E28/N30/R33/R38/K40, K28/N30/N33/Q38/A40, S28/N30/Y33/Q38/K40, T28/N30/R33/Q38/R40, Q28/N30/T33/Q38/R40, K28/N30/R33/T38/Q40, K28/N30/R33/T38/R40, Q28/N30/E33/D38/H40, R28/N30/Y33/N38/A40, Q28/N30/Y33/T38/R40, R28/N30/T33/R38/A40, H28/N30/Y33/D38/S40, Q28/N30/Y33/R38/A40, Q28/N30/Y33/A38/R40, S28/N30/Q33/A38/A40, Q28/N30/Y33/E38/K40, T28/N30/N33/Q38/R40, Q28/N30/Y33/R38/S40, K28/N30/R33/Q38/R40, Q28/N30/R33/A38/R40, Q28/N30/N33/Q38/R40, R28/N30/R33/E38/R40, K28/N30/R33/A38/R40, K28/N30/T33/A38/A40, K28/N30/R33/K38/A40, R28/N30/A33/K38/S40, K28/N30/R33/N38/A40, T28/N30/E33/S38/D40, R28/N30/N33/Q38/D40, R28/N30/R33/Y38/Q40, K28/N30/Y33/Q38/N40, K28/N30/R33/S38/S40, K28/N30/R33/Y38/A40, A28/N30/N33/R38/K40, K28/N30/R33/A38/T40, K28/N30/R33/N38/Q40, T28/N30/T33/Q38/R40, K28/N30/R33/Q38/Y40, Q28/N30/S33/R38/K40, R28/N30/Y33/Q38/S40, Q28/N30/R33/Q38/R40, K28/N30/R33/A38/Q40, A28/N30/R33/Q38/R40, K28/N30/R33/Q38/Q40, K28/N30/R33/Q38/A40, K28/N30/T33/A38/S40, K28/A30/H33/R38/S40, K28/H30/H33/R38/S40, K28/D30/N33/H38/S40, K28/E30/S33/R38/S40, K28/H30/T33/P38/S40, K28/G30/H33/Y38/S40, K28/A30/R33/Q38/S40, K28/S30/R33/G38/S40, K28/S30/H33/H38/S40, K28/N30/H33/R38/S40, K28/R30/R33/E38/S40, K28/D30/G33/H38/S40, K28/R30/H33/G38/S40, K28/A30/N33/Q38/S40, K28/D30/H33/K38/S40, K28/K30/H33/R38/S40, K28/Q30/N33/Q38/S40, K28/Q30/T33/Q38/S40, K28/G30/R33/Q38/S40 K28/R30/P33/G38/S40, K28/R30/G33/N38/S40, K28/N30/A33/Q38/S40, K28/N30/H33/N38/S40, K28/H30/H33/A38/S40, K28/R30/G33/S38/S40, K28/S30/R33/Q38/S40, K28/T30/D33/H38/S40, K28/H30/H33/Q38/S40, K28/A30/D33/H38/S40, K28/S30/H33/R38/S40, K28/N30/R33/A38/S40, K28/S30/H33/Q38/S40, K28/D30/A33/H38/S40, K28/N30/H33/E38/S40, K28/D30/R33/T38/S40, K28/D30/R33/S38/S40, K28/A30/H33/Q38/S40, K28/R30/G33/T38/S40, K28/N30/H33/S38/S40, K28/Q30/H33/Q38/S40, K28/N30/H33/G38/S40, K28/N30/N33/Q38/S40, K28/N30/D33/Q38/S40, K28/D30/R33/G38/S40, K28/N30/H33/A38/S40, K28/H30/M33/A38/S40, K28/S30/S33/H38/S40, K28/G30/V33/A38/S40, K28/S30/V33/Q38/S40, K28/D30/V33/H38/S40, R28/D30/V33/Q38/S40, K28/G30/V33/Q38/S40, K28/G30/V33/T38/S340, K28/G30/V33/H38/S40, K28/G30/V33/R38/S40, K28/G30/V33/G38/S40, R28/A30/V33/G38/S40, R28/D30/V33/R38/S40, R28/N30/V33/Q38/S40, and N28/T30/V33/D38/S40.

According to a more preferred embodiment, said I-CreI variant is a variant able to cleave at least a DNA target sequence which is not cleaved by the parent homing endonuclease (I*-Cre*I D75N), said variant having amino acid residues in positions 28, 30, 33, 38 and 40 respectively, which are selected from the group consisting of Q28/N30/Y33/K38/R40, R28/N30/K33/R38/Q40, Q28/N30/R33/R38/R40, Q28/N30/Y33/K38/K40, Q28/N30/T33/Q38/K40, Q28/N30/R33/R38/K40, K28/N30/T33/Q38/R40, S28/N30/R33/S38/R40, K28/N30/T33/R38/Q40, K28/N30/S33/R38/E40, S28/N30/Y33/R38/K40, K28/N30/S33/R38/D40, K28/N30/R33/E38/R40, K28/N30/S33/R38/S40, R28/N30/R33/D38/R40, A28/N30/S33/Q38/R40, Q28/N30/Y33/R38/K40, Q28/N30/K33/R38/T40, R28/N30/A33/Y38/Q40, K28/N30/R33/Q38/E40, N28/N30/S33/R38/K40, N28/N30/S33/R38/R40, S28/N30/R33/Q38/R40, Q28/N30/R33/Q38/K40, E28/N30/R33/R38/K40, K28/N30/N33/Q38/A40, S28/N30/Y33/Q38/K40, T28/N30/R33/Q38/R40, Q28/N30/T33/Q38/R40, K28/N30/R33/T38/Q40, K28/N30/R33/T38/R40, Q28/N30/E33/D38/H40, R28/N30/T33/R38/A40, H28/N30/Y33/D38/S40, S28/N30/Q33/A38/A40, K28/N30/R33/Q38/R40, Q28/N30/R33/A38/R40, R28/N30/R33/E38/R40, K28/N30/R33/A38/R40, K28/N30/T33/A38/A40, K28/N30/R33/K38/A40, K28/N30/R33/N38/A40, T28/N30/E33/S38/D40, R28/N30/R33/Y38/Q40, K28/N30/R33/S38/S40, K28/N30/R33/Y38/A40, A28/N30/N33/R38/K40, K28/N30/R33/A38/T40, K28/N30/R33/N38/Q40, T28/N30/T33/Q38/R40, K28/N30/R33/Q38/Y40, Q28/N30/S33/R38/K40, R28/N30/Y33/Q38/S40, Q28/N30/R33/Q38/R40, K28/N30/R33/A38/Q40, A28/N30/R33/Q38/R40, K28/N30/R33/Q381Q40, K28/N30/R33/Q38/A40, K28/N30/T33/A38/S40, K28/A30/H33/R38/S40, K28/H30/H33/R38/S40, K28/D30/N33/H38/S40, K28/E30/S33/R38/S40, K28/H30/T33/P38/S40, K28/G30/H33/Y38/S40, K28/A30/R33/Q38/S40, K28/S30/R33/G38/S40, K28/S30/H33/H38/S40, K28/N30/H33/R38/S40, K28/R30/R33/E38/S40, K28/D30/G33/H38/S40, K28/R30/H33/G38/S40, K28/A30/N33/Q38/S40, K28/D30/H33/K38/S40, K28/K30/H33/R38/S40, K28/Q30/N33/Q38/S40, K28/Q30/T33/Q38/S40, K28/G30/R33/Q38/S40 K28/R30/P33/G38/S40, K28/R30/G33/N38/S40, K28/N30/A33/Q38/S40, K28/N30/H33/N38/S40, K28/H30/H33/A38/S40, K28/R30/G33/S38/S40, K28/S30/R33/Q38/S40, K28/T30/D33/H38/S40, K28/H30/H33/Q38/S40, K28/A30/D33/H38/S40, K28/S30/H33/R38/S40, K28/N30/R33/A381S40, K28/S30/H33/Q38/S40, K28/D30/A33/H38/S40, K28/N30/H33/E38/S40, K28/D30/R33/T38/S40, K28/D30/R33/S38/S40, K28/A30/H33/Q38/S40, K28/R30/G33/T38/S40, K28/N30/H33/S38/S40, K28/Q30/H33/Q38/S40, K28/N30/H33/G38/S40, K28/N30/N33/Q38/S40, K28/N30/D33/Q38/S40, K28/D30/R33/G38/S40, K28/N30/H33/A38/S40, K28/H30/M33/A38/S40, K28/S30/S33/H38/S40, K28/G30/V33/A38/S40, K28/D30/V33/H38/S40, R28/D30/V33/Q38/S40, K28/G30/V33/T38/S340, K28/G30/V33/H38/S40, K28/G30/V33/R38/S40, and K28/G30/V33/G38/S40.

According to another more preferred embodiment, said I*-Cre*I variant is a variant able to cleave a DNA target sequence consisting of a mutant I*-Cre*I site wherein at least the a in position -8 and/or the t in position +8 has been replaced with a different nucleotide, said variant having amino acid residues in positions 28, 30, 33, 38 and 40 respectively, which are selected from the group consisting of: Q28/N30/Y33/K38/R40, R28/N30/K33/R38/Q40, Q28/N30/R33/R38/R40, Q28/N30/Y33/K38/K40, Q28/N30/T33/Q38/K40, Q28/N30/R33/R38/K40, K28/N30/T33/Q38/R40, S28/N30/R33/S38/R40, N28/N30/Y33/Q38/R40, K28/N30/S33/R38/E40, Q28/N30/N33/Q38/K40, S28/N30/Y33/R38/K40, K28/N30/S33/R38/D40, K28/N30/R33/E38/R40, K28/N30/S33/R38/S40, R28/N30/R33/D38/R40, A28/N30/S33/Q38/R40, Q28/N30/Y33/R38/K40, Q28/N30/K33/R38/T40, R28/N30/A33/Y38/Q40, K28/N30/R33/Q38/E40, N28/N30/S33/R38/K40, N28/N30/S33/R38/R40, Q28/N30/Y33/Q38/K40, Q28/N30/Y33/Q38/R40, S28/N30/R33/Q38/R40, Q28/N30/R33/Q38/K40, E28/N30/R33/R38/K40, K28/N30/N33/Q38/A40, S28/N30/Y33/Q38/K40, T28/N30/R33/Q38/R40, Q28/N30/T33/Q38/R40, K28/N30/R33/T38/R40, Q28/N30/E33/D38/H40, R28/N30/Y33/N38/A40, Q28/N30/Y33/T38/R40, R28/N30/T33/R38/A40, H28/N30/Y33/D38/S40, Q28/N30/Y33/R38/A40, Q28/N30/Y33/A38/R40, S28/N30/Q33/A38/A40, Q28/N30/Y33/E38/K40, T28/N30/N33/Q38/R40, Q28/N30/Y33/R38/S40, K28/N30/R33/Q38/R40, Q28/N30/R33/A38/R40, Q28/N30/N33/Q38/R40, R28/N30/R33/E38/R40, K28/N30/R33/A38/R40, K28/N30/T33/A38/A40, K28/N30/R33/K38/A40, R28/N30/A33/K38/S40, K28/N30/R33/N38/A40, T28/N30/E33/S38/D40, R28/N30/N33/Q38/D40, R28/N30/R33/Y38/Q40, K28/N30/Y33/Q38/N40, K28/N30/R33/S38/S40, K28/N30/R33/Y38/A40, A28/N30/N33/R38/K40, K28/N30/R33/A38/T40, T28/N30/T33/Q38/R40, K28/N30/R33/Q38/Y40, Q28/N30/S33/R38/K40, R28/N30/Y33/Q38/S40, Q28/N30/R33/Q38/R40, A28/N30/R33/Q38/R40, K28/N30/R33/Q38/Q40, K28/N30/R33/Q38/A40, K28/N30/T33/A38/S40, K28/A30/H33/R38/S40, K28/H30/H33/R38/S40, K28/D30/N33/H38/S40, K28/E30/S33/R38/S40, K28/H30/T33/P38/S40, K28/G30/H33/Y38/S40, K28/A30/R33/Q38/S40, K28/S30/R33/G38/S40, K28/S30/H33/H38/S40, K28/N30/H33/R38/S40, K28/R30/R33/E38/S40, K28/D30/G33/H38/S40, K28/R30/H33/G38/S40, K28/A30/N33/Q38/S40, K28/D30/H33/K38/S40, K28/K30/H33/R38/S40, K28/Q30/N33/Q38/S40, K28/Q30/T33/Q38/S40, K28/G30/R33/Q38/S40 K28/R30/P33/G38/S40, K28/R30/G33/N38/S40, K28/N30/A33/Q38/S40, K28/N30/H33/N38/S40, K28/H30/H33/A38/S40, K28/R30/G33/S38/S40, K28/S30/R33/Q38/S40, K28/T30/D33/H38/S40, K28/H30/H33/Q38/S40, K28/A30/D33/H38/S40, K28/S30/H33/R38/S40, K28/N30/R33/A38/S40, K28/S30/H33/Q38/S40, K28/D30/A33/H38/S40, K28/N30/H33/E38/S40, K28/D30/R33/T38/S40, K28/D30/R33/S38/S40, K28/A30/H33/Q38/S40, K28/R30/G33/T38/S40, K28/N30/H33/S38/S40, K28/Q30/H33/Q38/S40, K28/N30/H33/G38/S40, K28/N30/N33/Q38/S40, K28/N30/D33/Q38/S40, K28/D30/R33/G38/S40, K28/N30/H33/A38/S40, K28/H30/M33/A38/S40, K28/S30/S33/H38/S40, K28/G30/V33/A38/S40, K28/S30/V33/Q38/S40, K28/D30/V33/H38/S40, R28/D30/V33/Q38/S40, K28/G30/V33/Q38/S40 K28/G30/V33/T38/S40, K28/G30/V33/H38/S40, K28/G30/V33/R38/S40, K28/G30/V33/G38/S40, R28/A30/V33/G38/S40, R28/D30/V33/R38/S40, R28/N30/V33/Q38/S40, and N28/T30/V33/D38/S40.

According to yet another more preferred embodiment, said *I-CreI* variant is a variant able to cleave a DNA target sequence consisting of a mutant *I-CreI* site wherein at least the a in position -9 and/or the t in positions +9 has been replaced with a different nucleotide, said variant having amino acid residues in positions 28, 30, 33, 38 and 40 respectively, which are selected from the group consisting of: Q28/N30/Y33/K38/R40, R28/N30/K33/R38/Q40, Q28/N30/R33/R38/R40, Q28/N30/Y33/K38/K40, Q28/N30/T33/Q38/K40, Q28/N30/R33/R38/K40, K28/N30/T33/Q38/R40, S28/N30/R33/S38/R40, K28/N30/T33/R38/Q40, K28/N30/S33/R38/E40, S28/N30/Y33/R38/K40, K28/N30/S33/R38/D40, K28/N30/R33/E38/R40, K28/N30/S33/R38/S40, R28/N30/R33/D38/R40, Q28/N30/Y33/R38/K40, R28/N30/A33/Y38/Q40, N28/N30/S33/R38/K40, N28/N30/S33/R38/R40, E28/N30/R33/R38/K40, K28/N30/N33/Q38/A40, K28/N30/R33/T38/Q40, K28/N30/R33/T38/R40, Q28/N30/E33/D38/H40, R28/N30/T33/R38/A40, H28/N30/Y33/D38/S40, K28/N30/R33/Q38/R40, Q28/N30/R33/A38/R40, R28/N30/R33/E38/R40, K28/N30/R33/A38/R40, K28/N30/T33/A38/A40, K28/N30/R33/K38/A40, K28/N30/R33/N38/A40, R28/N30/R33/Y38/Q40, K28/N30/R33/S38/S40, A28/N30/N33/R38/K40, K28/N30/R33/A38/T40, K28/N30/R33/N38/Q40, T28/N30/T33/Q38/R40, K28/N30/R33/Q38/Y40, Q28/N30/S33/R38/K40, K28/N30/R33/A38/Q40, A28/N30/R33/Q38/R40, K28/N30/R33/Q38/A40, K28/N30/T33/A38/S40, K28/A30/H33/R38/S40, K28/H30/H33/R38/S40, K28/D30/N33/H38/S40, K28/E30/S33/R38/S40, K28/S30/R33/G38/S40, K28/S30/H33/H38/S40, K28/N30/H33/R38/S40, K28/R30/R33/E38/S40, K28/D30/G33/H38/S40, K28/D30/H33/K38/S40, K28/K30/H33/R38/S40, K28/Q30/N33/Q38/S40, K28/R30/G33/N38/S40, K28/N30/H33/N38/S40, K28/H30/H33/A38/S40, K28/R3O/G33/S38/S40, K28/T30/D33/H38/S40, K28/A30/D33/H38/S40, K28/S30/H33/R38/S40, K28/N30/R33/A38/S40, K28/D30/A33/H38/S40, K28/D30/R33/T38/S40, K28/D30/R33/S38/S40, K28/R30/G33/T38/S40, K28/N30/H33/S38/S40, K28/N30/H33/G38/S40, K28/N30/N33/Q38/S40, K28/D30/R33/G38/S40, K28/N30/H33/A38/S40, K28/H30/M33/A38/S40, K28/S30/S33/H38/S40, K28/G30/V33/A38/S40, K28/D30N33/H38/S40, K28/G30N33/T38/S340, K28/G30/V33/H38/S40, K28/G30/V33/R38/S40, and K28/G30/V33/G38/S40.
According to another advantageous embodiment of said I*-Cre*I variant, it comprises one or more additional mutation(s).

The residues which are mutated may advantageously be at positions contacting the DNA target sequence or interacting directly or indirectly with said DNA target. Preferably, said mutations are in positions selected from the group consisting of: 124, Q26, S32, Q44, R68, R70, D75,177, and T140. Preferably, said I-*Cre*I variant comprises one or more mutations selected from the group consisting of:
- the mutation of the isoleucine in position 24 in a valine (124V),
- the mutation of the arginine in position 70, in a serine (R70S), and
- the mutation of the aspartic acid in position 75, in an uncharged amino acid, preferably an asparagine (D75N) or a valine (D75V).

Furthermore, other residues may be mutated on the entire I*-Cre*I sequence, and in particular in the C-terminal half of I-*Cre*I (positions 80 to 163). The substitutions in the C-terminal half of I*-Cre*I are preferably in positions: 80, 82, 85, 86, 87, 94, 96, 100, 103, 114, 115, 117, 125, 129, 131, 132, 147, 151, 153, 154, 155, 157, 159 and 160 of I*-Cre*I*.*

In addition, said variant may include one or more residues inserted at the NH₂ terminus and/or COOH terminus. For example, a methionine residue is introduced at the NH₂ terminus, a tag (epitope or polyhistidine sequence) is introduced at the NH₂ terminus and/or COOH terminus; said tag is useful for the detection and/or the purification of said variant.

The I*-Cre*I variant of the invention may be an homodimer or an heterodimer.

According to another advantageous embodiment of said I*-Cre*I variant, it is an heterodimer comprising monomers from two different variants.

The present invention encompasses also a single-chain chimeric endonuclease comprising a monomer from a I*-Cre*I variant, as defined above.

The subject-matter of the present invention is also a polynucleotide fragment encoding a I*-Cre*I variant or a single-chain chimeric endonuclease derived from said variant, as defined above.

The subject-matter of the present invention is also a recombinant vector comprising at least one polynucleotide fragment encoding a variant or a single-chain chimeric endonuclease derived from said variant, as defined above. Said vector may comprise a polynucleotide fragment encoding one monomer of a homodimeric variant, two monomers or one monomer and one domain of a single-chain molecule. Alternatively, said vector may comprise two different polynucleotide fragments, each encoding one of the monomers of a heterodimeric variant.

One type of preferred vector is an episome, i.e., a nucleic acid capable of extra-chromosomal replication. Preferred vectors are those capable of autonomous replication and/or expression of nucleic acids to which they are linked. Vectors capable of directing the expression of genes to which they are operatively linked are referred to herein as "expression vectors".

A vector according to the present invention comprises, but is not limited to, a YAC (yeast artificial chromosome), a BAC (bacterial artificial), a baculovirus vector, a phage, a phagemid, a cosmid, a viral vector, a plasmid, a RNA vector or a linear or circular DNA or RNA molecule which may consist of chromosomal, non chromosomal, semi-synthetic or synthetic DNA. In general, expression vectors of utility in recombinant DNA techniques are often in the form of "plasmids" which refer generally to circular double-stranded DNA loops which, in their vector form are not bound to the chromosome. Large numbers of suitable vectors are known to those of skill in the art.

Viral vectors include retrovirus, adenovirus, parvovirus (e. g. adeno-associated viruses), coronavirus, negative strand RNA viruses such as orthomyxovirus (e. g., influenza virus), rhabdovirus (e. g., rabies and vesicular stomatitis virus), para-myxovirus (e. g. measles and Sendai), positive strand RNA viruses such as picor-navirus and alphavirus, and double-stranded DNA viruses including adenovirus, herpesvirus (e. g., Herpes Simplex virus types 1 and 2, Epstein-Barr virus, cytomegalovirus), and poxvirus (e. g., vaccinia, fowlpox and canarypox). Other viruses include Norwalk virus, togavirus, flavivirus, reoviruses, papovavirus, hepadnavirus, and hepatitis virus, for example.

Vectors can comprise selectable markers, for example: neomycin phosphotransferase, histidinol dehydrogenase, dihydrofolate reductase, hygromycin phosphotransferase, herpes simplex virus thymidine kinase, adenosine deaminase, glutamine synthetase, and hypoxanthine-guanine phosphoribosyl transferase for eukaryotic cell culture; TRP1 for S. *cerevisiae;* tetracycline, rifampicin or ampicillin resistance in E. *coli.*

Preferably said vectors are expression vectors, wherein the sequence(s) encoding the variant of the invention is placed under control of appropriate transcriptional and translational control elements to permit production or synthesis of said variant. Therefore, said polynucleotide is comprised in expression cassette. More particularly, the vector comprises a replication origin, a promoter operatively linked to said encoding polynucleotide, a ribosome-binding site, an RNA-splicing site (when genomic DNA is used), a polyadenylation site and a transcription termination site. It also can comprise an enhancer. Selection of the promoter will depend upon the cell in which the polypeptide is expressed. Preferably, when said variant is an heterodimer, the two polynucleotides encoding each of the monomers are included in one vector which is able to drive the expression of both polynucleotides, simultaneously.

According to another advantageous embodiment of said, vector, it includes a targeting construct comprising sequences sharing homologies with the region surrounding the DNA target sequence as defined above.

More preferably, said targeting DNA construct comprises:
a) sequences sharing homologies with the region surrounding the DNA target sequence as defined above, and
b) sequences to be introduced flanked by sequence as defined in a).

The invention also concerns a prokaryotic or eukaryotic host cell which is modified by a polynucleotide or a vector as defined above, preferably an expression vector.

The invention also concerns a non-human transgenic animal or a transgenic plant, characterized in that all or part of their cells are modified by a polynucleotide or a vector as defined above.

As used herein, a cell refers to a prokaryotic cell, such as a bacterial cell, or eukaryotic cell, such as an animal, plant or yeast cell.

The subject-matter of the present invention is also a composition comprising at least one I*-Cre*I variant, one single-chain chimeric endonuclease derived from said variant, one or two polynucleotide(s), preferably included in expression vector(s), as defined above.

In a preferred embodiment of said composition, it contains a targeting DNA construct comprising the sequence which repairs the site of interest flanked by sequences sharing homologies with the targeted locus.

The polynucleotide sequence(s) encoding the variant or the single-chain chimeric endonuclease derived from said variant as defined in the present invention, may be prepared by any method known by the man skilled in the art. For example, they are amplified from a cDNA template, by polymerase chain reaction with specific primers. Preferably the codons of said cDNA are chosen to favour the expression of said protein in the desired expression system.

The recombinant vector comprising said polynucleotides may be obtained and introduced in a host cell by the well-known recombinant DNA and genetic engineering techniques.

The variant of the invention is produced by expressing the polypeptide(s) as defined above; preferably said polypeptide(s) are expressed or co-expressed in a host cell modified by one or two expression vector(s), under conditions suitable for the expression or co-expression of the polypeptides, and the variant is recovered from the host cell culture.

The subject-matter of the present invention is also a method of genetic engineering comprising a step of double-strand nucleic acid breaking in a site of interest located on a vector comprising a DNA target as defined hereabove, by contacting said vector with a I*-Cre*I variant or a single-chain chimeric endonuclease comprising said variant as defined above, thereby inducing a homologous recombination with another vector presenting homology with the sequence surrounding the cleavage site of said variant.

The subject-matter of the present invention is also a method of genome engineering comprising the steps of: 1) double-strand breaking a genomic locus comprising at least one DNA target sequence as defined above, by contacting said target with a I-CreI variant or a single-chain chimeric endonuclease comprising said variant as defined above; 2) maintaining said broken genomic locus under conditions appropriate for homologous recombination with a targeting DNA construct comprising the sequence to be introduced in said locus, flanked by sequences sharing homologies with the targeted locus.

The subject-matter of the present invention is also a method of genome engineering, characterized in that it comprises the following steps: 1) double-strand breaking a genomic locus comprising at least one DNA target sequence as defined above, by contacting said cleavage site with a I-CreI variant or a single-chain chimeric endonuclease comprising said variant as defined above; 2) maintaining said broken genomic locus under conditions appropriate for homologous recombination with chromosomal DNA sharing homologies to regions surrounding the cleavage site.

The subject-matter of the present invention is also the use of a I*-Cre*I endonuclease variant obtainable by the method as described above for molecular biology, *in vivo or in vitro* genetic engineering, and *in vivo or in vitro* genome engineering for non-therapeutic purposes, for cleaving a DNA target sequence as defined above.

Molecular biology includes with no limitations, DNA restriction and DNA mapping. Genetic and genome engineering for non therapeutic purposes include for example (i) gene targeting of specific loci in cell packaging lines for protein production, (ii) gene targeting of specific loci in crop plants, for strain improvements and metabolic engineering, (iii) targeted recombination for the removal of markers in genetically modified crop plants, (iv) targeted recombination for the removal of markers in genetically modified microorganism strains (for antibiotic production for example).

According to an advantageous embodiment of said use, it is for inducing a double-strand break in a site of interest comprising a DNA target sequence cleaved by a variant as defined above, thereby inducing a DNA recombination event, a DNA loss or cell death.

In a particular embodiment, an I*-Cre*I variant having an arginine (R) or a lysine (K) in position 38 is used for cleaving a DNA target comprising a guanine in position -9 or a cytosine in position +9.

According to the invention, said double-strand break is for: repairing a specific sequence, modifying a specific sequence, restoring a functional gene in place of a mutated one, attenuating or activating an endogenous gene of interest, introducing a mutation into a site of interest, introducing an exogenous gene or a part thereof, inactivating or detecting an endogenous gene or a part thereof, translocating a chromosomal arm, or leaving the DNA unrepaired and degraded.

The subject-matter of the present invention is also the use of at least one I*-Cre*I variant as defined above, for the preparation of a medicament for preventing, improving or curing a genetic disease in an individual in need thereof, said medicament being administrated by any means to said individual.

The subject-matter of the present invention is also a method for preventing, improving or curing a genetic disease in an individual in need thereof, said method comprising at least the step of administering to said individual a composition as defined above, by any means.

In a particular embodiment, an I*-Cre*I variant having an arginine (R) or a lysine (K) in position 38 is used for cleaving a genomic DNA target comprising a guanine in position -9 or a cytosine in position +9.

The subject-matter of the present invention is also the use of at least one I*-Cre*I variant as defined above, for the preparation of a medicament for preventing, improving or curing a disease caused by an infectious agent that presents a DNA intermediate, in an individual in need thereof, said medicament being administrated by any means to said individual.

The subject-matter of the present invention is also a method for preventing, improving or curing a disease caused by an infectious agent that presents a DNA intermediate, in an individual in need thereof, said method comprising at least the step of administering to said individual a composition as defined above, by any means.

The subject-matter of the present invention is also the use of at least one I*-Cre*I variant, as defined above, *in vitro,* for inhibiting the propagation, inactivating or deleting an infectious agent that presents a DNA intermediate, in biological derived products or products intended for biological uses or for disinfecting an object.

The subject matter of the present invention is also a method for decontaminating a product or a material from an infectious agent that presents a DNA intermediate, said method comprising at least the step of contacting a biological derived product, a product intended for biological use or an object, with a composition as defined above, for a time sufficient to inhibit the propagation, inactivate or delete said infectious agent.

In a particular embodiment, an I*-Cre*I variant having an arginine (R) or a lysine (K) in position 38 is used for cleaving a DNA target from said infectious agent that comprises a guanine in position -9 or a cytosine in position +9.

In another particular embodiment, said infectious agent is a virus. For example said virus is an adenovirus (Ad11, Ad21), herpesvirus (HSV, VZV, EBV, CMV, herpesvirus 6, 7 or 8), hepadnavirus (HBV), papovavirus (HPV), poxvirus or retrovirus (HTLV, HIV).

The subject-matter of the present invention is also the use of at least one I*-Cre*I variant, as defined above, as a scaffold for making other meganucleases. For example a second round of mutagenesis and selection/screening can be performed on said I*-Cre*I variant, for the purpose of making novel, second generation homing endonucleases.

According to another advantageous embodiment of said uses, said I-CreI variant is associated with a targeting DNA construct as defined above.

According to another advantageous embodiment of said uses, said I-CreI variant has amino acid residues in positions 28, 30, 33, 38 and 40 respectively, which are selected from the group consisting of: KNSQS, KNRQS, KNTQS, KNHQS.

The use of the I*-Cre*I meganuclease variant and the methods of using said I*-Cre*I meganuclease variant according to the present invention include also the use of the single-chain chimeric endonuclease derived from said variant, the polynucleotide(s), vector, cell, transgenic plant or non-human transgenic mammal encoding said variant or single-chain chimeric endonuclease, as defined above.

In addition to the preceding features, the invention further comprises other features which will emerge from the description which follows, which refers to examples illustrating the I*-Cre*I homing endonuclease variants and their uses according to the invention, as well as to the appended drawings in which:
- figure 1 represents the DNA targets. A. Two palindromic I*-Cre*I targets derived from the natural I*-Cre*I homing site. The I*-Cre*I natural target contains two palindromes, boxed in grey: the -8 to -12 and +8 to +12 nucleotides on one hand, and the -5 to -3 and +3 to +5 nucleotide on another hand. From the natural target, called here C1234 (SEQ ID NO: 1), can be derived to palindromic sequences, C1221 1 and C4334 (SEQ ID NO: 2, 3). Both are cut by I*-Cre*I*,* in *vitro* and in yeast. B. The 64 DNA targets. The 64 targets are derived from C1221 (SEQ ID NO: 4 to 67). They correspond to all the perfect 24 bp palindromes resulting from substitutions at positions -10, -9, -8, +8, +9 and +10.
- figure 2 illustrates the principle of the yeast screening assay. A strain expressing the meganuclease to be assayed (MEGA), marked with the *LEU2*

### A) Material and methods

### a) Construction of mutant libraries

I*-Cre*I wt (I*-Cre*I D75), I*-Cre*I D75N (I*-Cre*I N75) and I*-Cre*I S70 N75 open reading frames were synthesized, as described previously (Epinat et al., N.A.R., 2003, 31, 2952-2962). Combinatorial libraries were derived from the I*-Cre*I N75, I*-Cre*I D75 or I*-Cre*I S70 N75 scaffold, by replacing two or three different combinations of residues, potentially involved in the interactions with the bases in positions ± 8 to 10 of one DNA target half-site. The diversity of the meganuclease libraries was generated by PCR using degenerated primers harboring a unique degenerated codon at each of the selected positions.

Mutation D75N was introduced by replacing codon 75 with aac. Then, three codons at positions N30, Y33 and Q38 (Ulib4 library) or K28, N30 and Q38 (Ulib5 library) were replaced by a degenerated codon VVK (18 codons) coding for 12 different amino acids: A,D,E,G,H,K,N,P,Q,R,S,T). In consequence, the maximal (theoretical) diversity of these protein libraries was 12³ or 1728. However, in terms of nucleic acids, the diversity was 18³ or 5832.

In Lib4, ordered from BIOMETHODES, an arginine in position 70 of the I*-Cre*I N75 scaffold was first replaced with a serine (R70S). Then positions 28, 33, 38 and 40 were randomized. The regular amino acids (K28, Y33, Q38 and S40) were replaced with one out of 10 amino acids (A,D,E,K,N,Q,R,S,T,Y). The resulting library has a theoretical complexity of 10000 in terms of proteins.

In addition, small libraries of complexity 225 (15²) resulting from the randomization of only two positions were constructed in an I*-Cre*I N75 or I-Crel D75 scaffold, using NVK degenerate codon (24 codons, amino acids ACDEGHKNPQRSTWY).

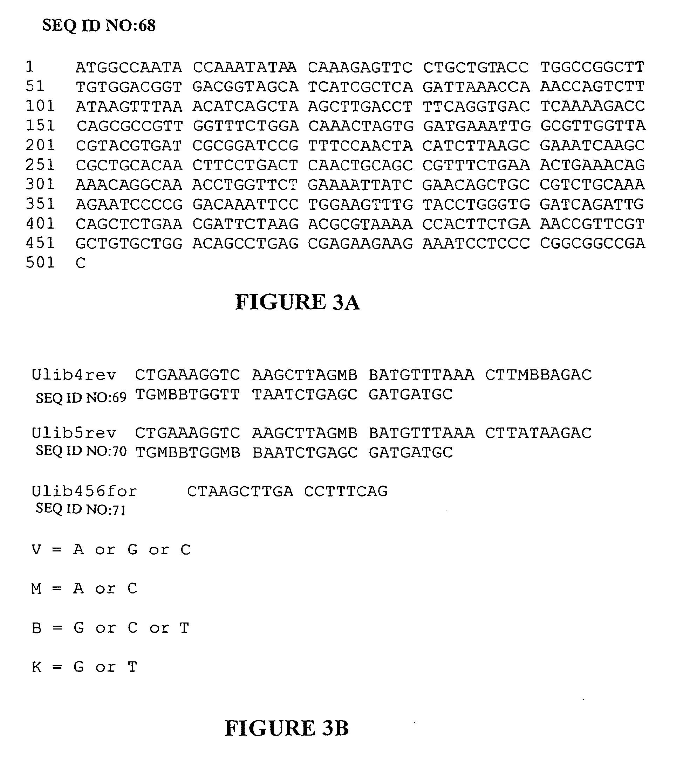

Fragments carrying combinations of the desired mutations were obtained by PCR, using a pair of degenerated primers coding for 10, 12 or 15 different amino acids, and as DNA template, the I*-Cre*I N75 (Figure 3A), I*-Cre*I D75 or I*-Cre*I S70 N75 open reading frames (ORF). For example, figure 3B illustrates the two pair of primers (Ulib456for and Ulib4rev; Ulib456for and Ulib5rev) used to generate the Ulib4 and Ulib5 libraries, respectively. The corresponding PCR products were cloned gene, is mated with a strain harboring a reporter plasmid containing the chosen target, marked by the *TRPI* gene. The target is flanked by overlapping truncated LacZ genes (LAC and ACZ). In diploids (*LEU2 TRP 1*)*,* cleavage of the target site by the meganuclease induces homologous recombination between the two lacZ repeats, resulting in a functional beta-galactosidase gene, that can be monitored by X-Gal staining.
- figure 3 represents the sequences of the I*-Cre*l N75 scaffold protein and degenerated primers used for the Ulib4 and Ulib5 libraries construction. A. The scaffolf (SEQ ID NO: 68) is the I-C*re*I ORF including the D75N codon substitution and three additional codons (AAD) at the 3' end. B. Primers (SEQ ID NO: 69, 70, 71),

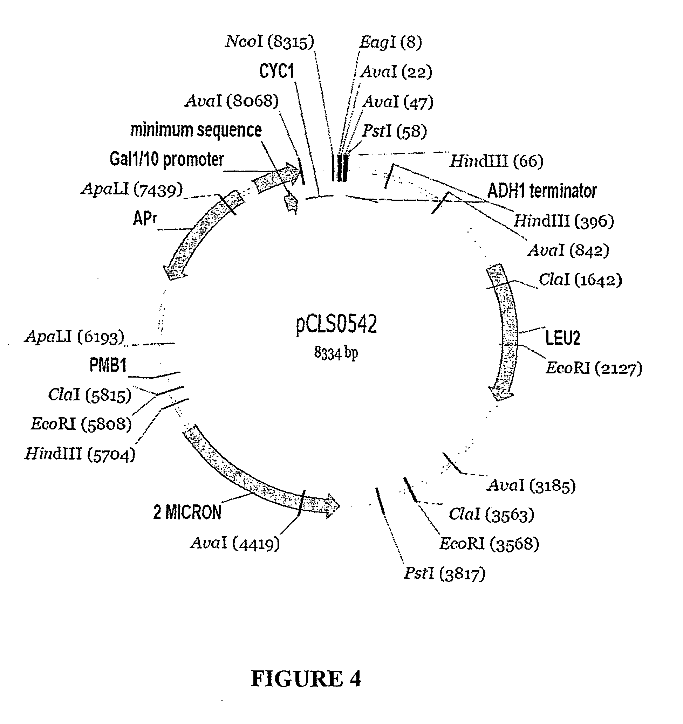

- figure 4 represents the pCLS0542 meganuclease expression vector. pCLS0542 is a 2 micron-based replicative vector marked with a LEU2 auxotrophic gene, and an inducible Ga110 promoter for driving the expression of the *I-Crel* variants,

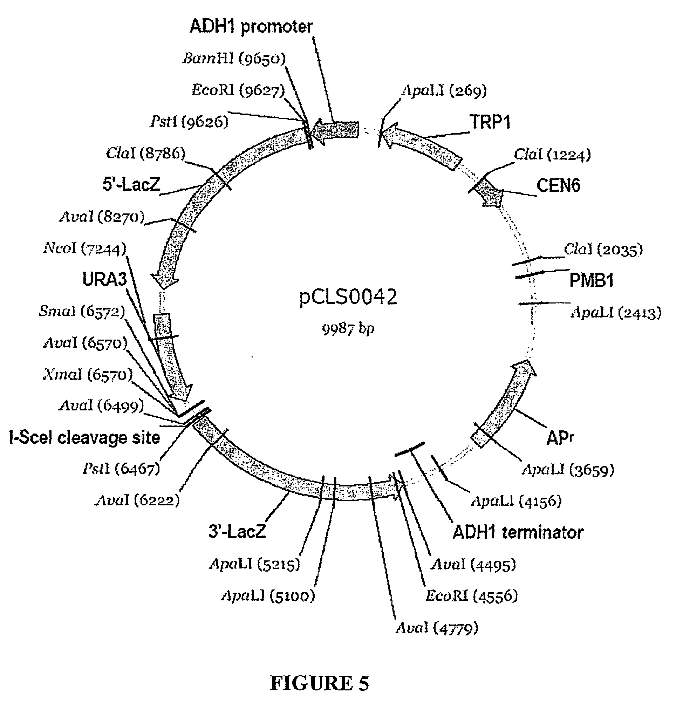

- figure 5 represents the pCLS0042 reporter vector. The reporter vector is marked by TRP1 and URA3. The LacZ tandem repeats share 800 bp of homology, and are separated by 1.3 kb of DNA. They are surrounded by ADH promoter and terminator sequences. Target sites are cloned into the *Sma*I site.

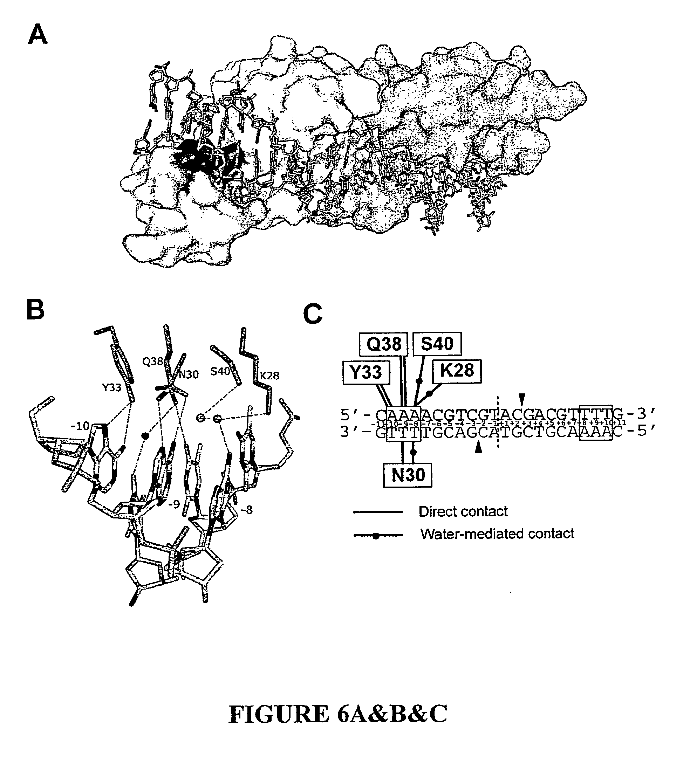

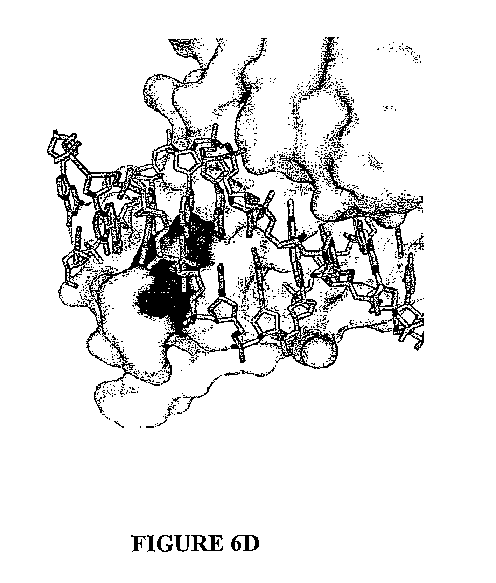

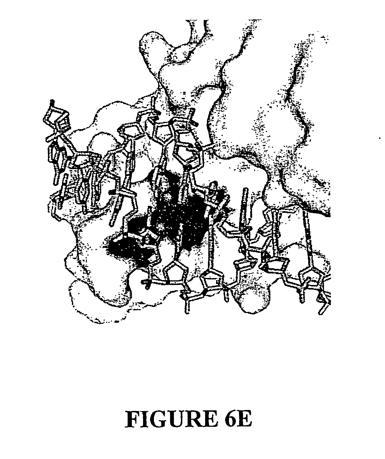

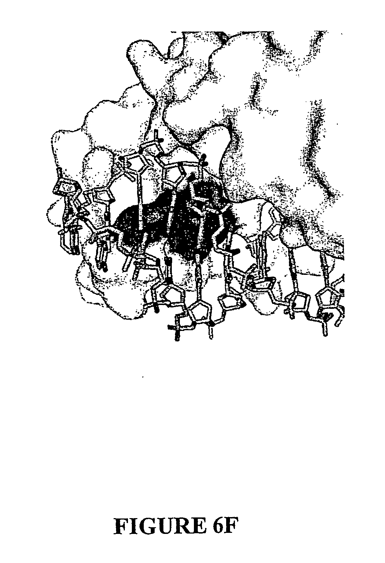

- figure 6 illustrates the rationale of the I-*Cre*I variants libraries. A. Structure of I-CreI homodimer bound to its DNA target, according to Chevalier et al., J. Mol. Biol., 2003, 329, 253-269, and localization of the area of the binding interface chosen for randomization in this study; residues 28, 30, 33, 38, and 40 are labelled in black on the monomer on the left. B. Zoom showing residues 28, 30, 33, 38 and 40 chosen for randomization. C. Summary of I-CreI/DNA interaction in the external region of the I-CreI DNA target (in black in figure 6A). The target represented, C1221 (SEQ ID NO: 3), is a palindromic target cleaved by I*-Cre*I (Chevalier *et al.,* 2003, precited). Only base specific contacts are indicated. The 10NNN region (±8, ±9, ±10 nucleotides, in black on Figure 6A) of the target is boxed. D. Position of residues randomized in Ulib4 (30, 33, 38). E. Position of residues randomized in Ulib5 (28, 30, 38). F. Position of residues randomized in Lib4 (28, 33, 38, 40).

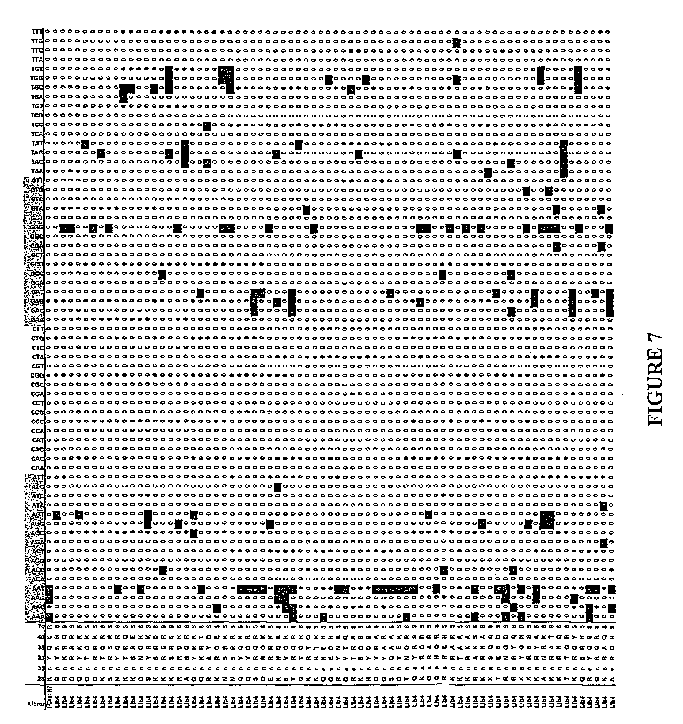

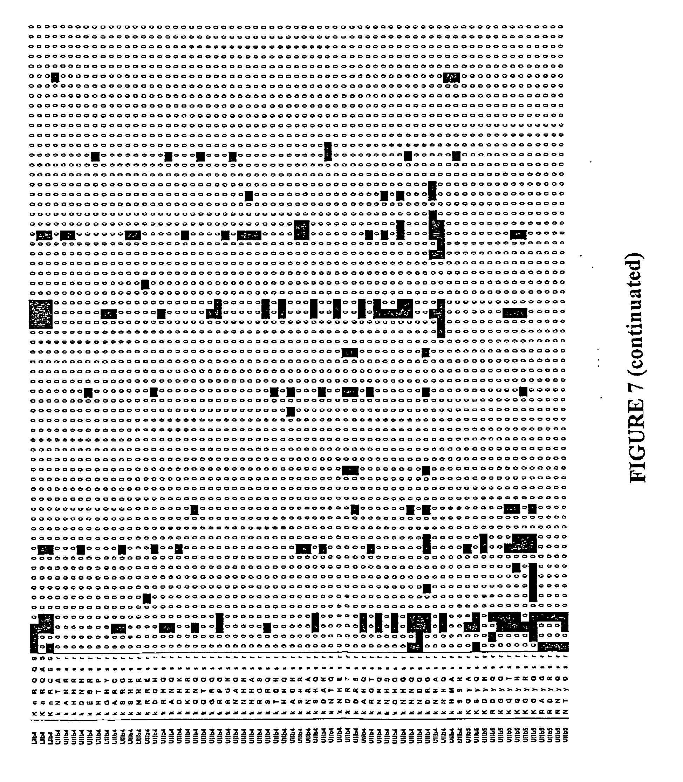

- figure 7 represents the cleavage patterns of 141 I-CreI variants cleaving 37 novel DNA targets. For each of the I-*Cre*I variants obtained after screening, and defined by residues in position 28, 30, 33, 38, 40, 70 and 75, cleavage was monitored in yeast with the 64 targets described in Figure 1B. Targets are designated by three letters, corresponding to the nucleotides in position -10, -9 and -8. For example GGG corresponds to the tcgggacgtcgtacgacgtcccga target (SEQ ID NO:4; see Figure 1). Values correspond to the intensity of the cleavage, evaluated by an appropriate software after scanning of the filter.

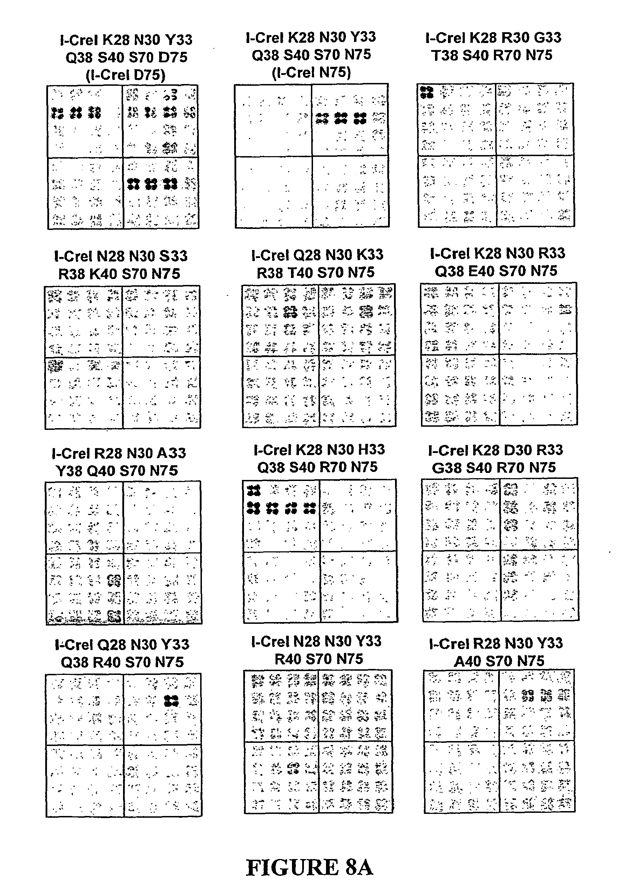

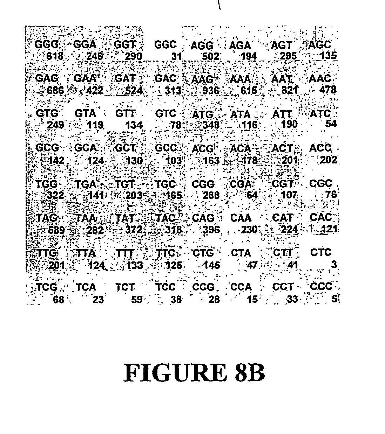

- figure 8 illustrates examples of patterns and the numbers of mutants cleaving each target. A. Examples of profiling. Each novel endonuclease is profiled in yeast on a series of 64 palindromic targets described in figure 1B, differing from the sequence shown in figure 1A, at positions ±8, ±9 and ±10. These targets are arrayed as in Figure 8B. Each target sequence is named after the -10,-9,-8 triplet (10NNN). For example GGG corresponds to the tcgggacgtcgtacgacgtcccga target (SEQ ID NO:4; see Figure 1 B). Meganucleases are tested 4 times against the 64 targets. Targets cleaved by I*-Cre*I (D75), I*-Cre*I N75 or ten derived variants are visualised by black or grey spots. B. Numbers of mutants cleaving each target, and average intensity of cleavage. Each sequence is named after the -10,-9,-8 triplet (10NNN). The number of proteins cleaving each target is shown below, and the level of grey coloration is proportional to the average signal intensity obtained with these cutters in yeast.

### Example 1: Functional endonucleases with new specificity towards nucleotides ±8, ±9, and ±10 (10NNN)

The method for producing meganuclease variants and the assays based on cleavage-induced recombination in yeast cells, which are used for screening variants with altered specificity, are described in the International PCT Application WO 2004/067736 and Epinat et al., N.A.R., 2003, 31, 2952-2962. These assays result in a functional LacZ reporter gene which can be monitored by standard methods (Figure 2).

### A) Material and methods

### a) Construction of mutant libraries

I*-Cre*I wt (I*-Cre*I D75), I*-Cre*I D75N (I*-Cre*I N75) and I*-Cre*I S70 N75 open reading frames were synthesized, as described previously (Epinat et al., N.A.R., 2003, 31, 2952-2962). Combinatorial libraries were derived from the I*-Cre*I N75, I*-Cre*I D75 or I*-Cre*I S70 N75 scaffold, by replacing two or three different combinations of residues, potentially involved in the interactions with the bases in positions ± 8 to 10 of one DNA target half-site. The diversity of the meganuclease libraries was generated by PCR using degenerated primers harboring a unique degenerated codon at each of the selected positions.

Mutation D75N was introduced by replacing codon 75 with aac. Then, three codons at positions N30, Y33 and Q38 (Ulib4 library) or K28, N30 and Q38 (Ulib5 library) were replaced by a degenerated codon VVK (18 codons) coding for 12 different amino acids : A,D,E,GH,K,N,P,Q,R,S,T). In consequence, the maximal (theoretical) diversity of these potein libraries was 12³ or 1728. However, in terms of nucleic acids, the diversity was 18³ or 5832.

In Lib4, ordered from BIOMETHODES, an arginine in position 70 of the I*-Cre*I N75 scaffold was first replaced with a serine (R70S). Then positions 28, 33, 38 and 40 were randomized. The regular amino acids (K28, Y33, Q38 and S40) were replaced with one out of 10 amino acids (A,D,E,K,N,Q,R,S,T,Y). The resulting library has a theoretical complexity of 10000 in terms of proteins.

In addition, small libraries of complexity 225 (15²) resulting from the randomization of only two positions were constructed in an I*-Cre*I N75 or I*-Cre*I D75 scaffold, using NVK degenerate codon (24 codons, amino acids ACDEGHKNPQRSTWY).

Fragments carrying combinations of the desired mutations were obtained by PCR, using a pair of degenerated primers coding for 10, 12 or 15 different amino acids, and as DNA template, the I*-Cre*I N75 (figure 3A), I*-Cre*I D75 or I*-Cre*I S70 N75 open reading frames (ORF). For example, figure 3B illustrates the two pair of primers (Ulib456for and Ulib4rev; Ulib456for and Ulib5rev) used to generate the Ulib4 and Ulib5 libraeries, respectively. The corresponding PCR products were cloned back into the I*-Cre*I N75 or I*-Cre*ID75 ORF, in the yeast replicative expression vector pCLS0542 (Epinat *et al.,* precited; Figure 4), carrying a *LEU2* auxotrophic marker gene. In this 2 micron-based replicative vector, I-*Cre*I variants are under the control of a galactose inducible promoter.

### b) Construction of target clones

The C1221 twenty-four bp palindrome (tcaaaacgtcgtacgacgttttga, SEQ ID NO: 3) is a repeat of the half-site of the nearly palindromic natural I-*Cre*I target (tcaaaacgtcgtgagacagtttgg, SEQ ID NO: 1). C1221 is cleaved as efficiently as the I*-Cre*I natural target *in vitro* and *ex vivo* in both yeast and mammalian cells. The 64 palindromic targets were derived as follows: 64 pairs of oligonucleotides (ggcatacaagtttcnnnacgtcgtacgacgtnnngacaatcgtctgtca (SEQ ID NO: 72) and reverse complementary sequences) were ordered form Sigma, annealed and cloned into pGEM-T Easy (PROMEGA) in the same orientation. Next, a 400 bp *Pvu*II fragment was excised and cloned into the yeast vector pFL39-ADH-LACURAZ, also called pCLS0042, described previously (Epinat *et al.,* precited, Figure 5), resulting in 64 yeast reporter vectors (target plasmids).

### c) Yeast strains

The three libraries of meganuclease expression variants were transformed into the *leu2* mutant haploid yeast strain FYC2-6A: *MATalpha, trp1 Δ63, leu2*Δ*1, his3*Δ*200.* A classical chemical/heat choc protocol that routinely gives us 10⁶ independent transformants per µg of DNA derived from (Gietz and Woods, Methods Enzymol., 2002, 350, 87-96), was used for transformation. Individual transformant (Leu+) clones were individually picked in 96 wells microplates.
The 64 target plasmids were transformed using the same protocol, into the haploid yeast strain FYBL2-7B: *MATa, ura3*Δ*851, trpl*Δ*63, leu2*Δ*1, lys2*Δ*202,* resulting in 64 tester strains.

### d) Mating of meganuclease expressing clones and screening in yeast

Meganuclease expressing clones were mated with each of the 64 target strains, and diploids were tested for beta-galactosidase activity, by using the screening assay illustrated on figure 2.

I-Crel variant clones as well as yeast reporter strains were stocked in glycerol (20%) and replicated in novel microplates. Mating was performed using a colony gridder (QpixII, GENETIX). Mutants were spotted on nylon filters covering YPD plates, using a high density (about 20 spots/cm²). A second spotting process was performed on the same filters to spot a second layer consisting of 64 different reporter-harboring yeast strains for each variant. Membranes were placed on solid agarose YEPD rich medium, and incubated at 30°C for one night, to allow mating. Next, filters were transferred to synthetic medium, lacking leucine and tryptophan, with galactose (2 %) as a carbon source (and with G418 for coexpression experiments), and incubated for five days at 37°C, to select for diploids, allow for meganuclease expression, reporter plasmid cleavage and recombination, and expression of beta-galactosidase. After 5 days, filters were placed on solid agarose medium with 0.02 % X-Gal in 0.5 M sodium phosphate buffer, pH 7.0, 0.1 % SDS, 6 % dimethyl formamide (DMF), 7 mM β-mercaptoethanol, 1% agarose, and incubated at 37°C, to monitor β-galactosidase activity. After two days of incubation, positive clones were identified by scanning. The β-galactosidase activity of the clones was quantified using an appropriate software.

The clones showing an activity against at least one target were isolated (first screening). The spotting density was then reduced to 4 spots/cm² and each positive clone was tested against the 64 reporter strains in quadruplicate, thereby creating complete profiles (secondary screening).

### e) Sequence

The open reading frame (ORF) of positive clones identified during the first and/or secondary screening in yeast was amplified by PCR on yeast colonies using primers: PCR-Gal10-F (gcaactttagtgctgacacatacagg, SEQ ID NO: 73) and PCR-Gal10-R (acaaccttgattgcagacttgacc, SEQ ID NO: 74) from PROLIGO. Briefly, yeast colony is picked and resuspended in 100 µl of LGlu liquid medium and cultures overnight. After centrifugation, yeast pellet is resuspended in 10 µl of sterile water and used to perform PCR reaction in a final volume of 50 µl containing 1.5 µl of each specific primers (100 pmol/µl). The PCR conditions were one cycle of denaturation for 10 minutes at 94 °C, 35 cycles of denaturation for 30s at 94°C, annealing for 1 min at '55°C, extension for 1.5 min at 72 °C, and a final extension for 5 min. The resulting PCR products were then sequenced.

### f) Structure analyses

All analyses of protein structures were realized using Pymol. The structures from I-CreI correspond to pdb entry lg9y. Residue numbering in the text always refer to these structures, except for residues in the second I-*Cre*I protein domain of the homodimer where residue numbers were set as for the first domain.

### B) Results

I-*Cre*I is a dimeric homing endonuclease that cleaves a 22 bp pseudo-palindromic target. Analysis of I-*Cre*I structure bound to its natural target has shown that in each monomer, eight residues establish direct interactions with seven bases (Jurica *et al.,* 1998, precited). According to these structural data, the bases of the nucleotides in positions ± 8 to 10 establish direct contacts with I-*Cre*l amino-acids N30, Y33, Q38 and indirect contacts with I-Crel amino-acids K28 and S40 (Figure 6A, 6B, 6C). Thus, novel proteins with mutations in positions 30, 33 and 38 could display novel cleavage profiles with the 64 targets resulting from substitutions in positions ± 8, ± 9 and ± 10 of a palindromic target cleaved by I-*Cre*I (10NNN target). In addition, mutations might alter the number and positions of the residues involved in direct contact with the DNA bases. More specifically, positions other than 30, 33, 38, but located in the close vicinity on the folded protein, could be involved in the interaction with the same base pairs.

An exhaustive protein library vs. target library approach was undertaken to engineer locally this part of the DNA binding interface. Randomization of 5 amino acids positions would lead to a theoretical diversity of 20⁵ = 3.2x 10⁶. However, libraries with lower diversity were generated by randomizing 2, 3 or 4 residues at a time, resulting in a diversity of 225 (15²), 1728 (12³) or 10,000 (10⁴). This strategy allowed an extensive screening of each of these libraries against the 64 palindromic 10NNN DNA targets using a yeast based assay described previously (Epinat *et al*., 2003, precited and International PCT Application WO 2004/067736) and whose principle is described in Figure 2.

First, the I-*Cre*I scaffold was mutated from D75 to N. The D75N mutation did not affect the protein structure, but decreased the toxicity of I-*Cre*I in overexpression experiments.

Next the Ulib4 library was constructed : residues 30, 33 and 38 (Figure 6A-C and 6D), were randomized, and the regular amino acids (N30, Y33, and Q38) replaced with one out of 12 amino acids (A,D,E,G,H,KN,P,Q,R,S,T). The resulting library has a complexity of 1728 in terms of protein (5832 in terms of nucleic acids).

Then, two other libraries were constructed : Ulib5 and Lib4. In Ulib5, residues 28, 30 and 38 (Figure 6A-C and 6E), were randomized, and the regular amino acids (K28, N30, and Q38) replaced with one out of 12 amino acids (ADEGHKNPQRST). The resulting library has a complexity of 1728 in terms of protein (5832 in terms of nucleic acids). In Lib4, an Arginine in position 70 was first replaced with a Serine. Then, positions 28,33,38 and 40 (Figure 6A-C and 6F) were randomized, and the regular amino acids (K28, Y33, Q38 and S40) replaced with one out of 10 amino acids (A,D,E,K,N,Q,R,S,T,Y). The resulting library has a complexity of 10000 in terms of proteins.

In a primary screening experiment, 20000 clones from Ulib4, 10000 clones from Ulib5 and 20000 clones from Lib4 were mated with each one of the 64 tester strains, and diploids were tested for beta-galactosidase activity. All clones displaying cleavage activity with at least one out of the 64 targets were tested in a second round of screening against the 64 targets, in quadriplate, and each cleavage profile was established, as shown on Figure 8. Then, meganuclease ORF were amplified from each strain by PCR, and sequenced.

After secondary screening and sequencing of positives over the entire coding region, a total of 1484 unique mutants were isolated showing a cleavage activity against at least one target. Different patterns could be observed. Figure 7 illustrates 37 novel targets cleaved by a collection of 141 variants, including 34 targets which are not cleaved by I-*Cre*I and 3 targets which are cleaved by I-*Cre*I (aag, aat and aac). Twelve examples of profile, including I-*Cre*I N75 and I-*Cre*I D75 are shown on Figure 8A. Some of these new profiles shared some similarity with the wild type scaffold whereas many others were totally different. Homing endonucleases can usually accommodate some degeneracy in their target sequences, and the I-*Cre*I and I-*CreI* N75 proteins themselves cleave a series of sixteen and three targets, respectively. Cleavage degeneracy was found for many of the novel endonucleases, with an average of 9.9 cleaved targets per mutant (standard deviation: 11). However, among the 1484 mutants identified, 219 (15%) were found to cleave only one DNA target, 179 (12 %) cleave two, and 169 (11 %) and 120 (8 %) were able to cleave 3 and 4 targets respectively. Thus, irrespective of their preferred target, a significant number of I-*Cre*I derivatives display a specificity level that is similar if not higher than that of the I-CreI N75 mutant (three 10NNN target sequences cleaved), or I-*Cre*I (sixteen 10NNN target sequences cleaved). Also, the majority of the mutants isolated for altered specificity for 10NNN sequences no longer cleave the original C1221 target sequence described in Figure 6C (61% and 59%, respectively).

Altogether, this large collection of mutants allowed the targeting of all of the 64 possible DNA sequences differing at positions ±10, ±9, and ±8 (Figure 8B). However, there were huge variations in the numbers of mutants cleaving each target (Figure 8B), these numbers ranged from 3 to 936, with an average of 228.5 (standard deviation: 201.5). Cleavage was frequently observed for targets with a guanine in ±8 or an adenine in ±9, whereas a cytosine in ±10 or ±8 was correlated with low numbers of cleavers. In addition, all targets were not cleaved with the same efficiency. Since significant variations of signal could be observed for a same target, depending on the mutant (compare cleavage efficiencies for the wild type 10AAA target in Figure 8B, for example), an average cleavage efficiency was measured for each target as previously reported (Amould et al., J. Mol. Biol., 2006, 355, 443-458). These average efficiencies are represented by grey levels on Figure 8B. Analysis of the results show a clear correlation between this average efficiency and the numbers of cleavers, with the most frequently cut target being also the most efficiently cut (compare for example 10TCN, 1OCTN and 10CCN targets with 10GAN, 10AAN and 10TAN in Figure 8B).

Thus, hundreds of novel variants were obtained, including mutants with novel substrate specificity ; these variants can keep high levels of activity and the specificity of the novel proteins can be even narrower than that of the wild-type protein for its target.

### Example 2: Statistical analysis of interactions between I-CreI variants and their targets

### A) Material and methods

Hierarchical clustering was used to establish potential correlations between specific protein residues and target bases, as previously described (Arnould et al., J. Mol. Biol., 2006, 355, 443-458). Clustering was done on the quantitative data from the secondary screening, using hclust from the R package. Variants were clustered using standard hierarchical clustering with Euclidean distance and Ward's method (Ward, J.H., American Statist. Assoc., 1963, 58, 236-244). Mutant dendrogram was cut at the height of 17 to define the clusters. For the analysis, cumulated intensities of cleavage of a target within a cluster was calculated as the sum of the cleavage intensities of all cluster's mutants with this target, normalized to the sum of the cleavage intensities of all cluster's mutants with all targets.

### B) Results

Ten different mutant clusters were identified (Table I).

**Table I: Cluster analysis**

| cluster | preferred targets | | nucleotide | | nucleotide | | nucleotide | | preferred amino acids (%) | | | | |
|---|---|---|---|---|---|---|---|---|---|---|---|---|---|
| (effectif) | 10NNN | (%) | -10 (%) | | -9 (%) | | -8 (%) | | 28 | 30 | 33 | 38 | 40 |
| | | | | | | | | | K | | | | |
| 1 | GGG | 7,1 | A | 27,0 | A | 45,9 | A | 21,3 | 100 | N45.5 | H | Q | S |
| (44) | GAG | 6,9 | C | 4,7 | C | 14,5 | C | 14,7 | 100 | 45,5 | 38,6 | 70,5 | 86,4 |
| | GAT | 6,4 | G | 63,0 | G | 27,9 | G | 37,0 | | K | R | | |
| | | S=20.4 | T | 5,3 | T | 11,8 | T | 27,0 | | 15,9 | 25,0 | | |
| | | | | | | | | | | R | | | |
| | | | | | | | | | | 15,9 | | | |
| 2 | AAG | 6,1 | A | 33,4 | A | 52,2 | A | 20,4 | K | N | G | Q | S |
| (82) | TAG | 5,6 | C | 11,7 | C | 9,4 | C | 13,8 | 100 | 64,6 | 23,2 | 68,3 | 79,3 |
| | GAG | 5,2 | G | 23,7 | G | 19,9 | G | 41,7 | | | | | |
| | | S=16.9 | T | 31,2 | T | 18,5 | T | 24,0 | | | | | |
| 3 | TAG | 4,5 | A | 24,7 | A | 45,2 | A | 19,5 | K | N | T | Q | S |
| (36) | TAC | 4,4 | C | 13,9 | C | 6,7 | C | 16,2 | 100 | 75,0 | 61,1 | 86,1 | 75,0 |
| | TGG | 4,3 | G | 15,8 | G | 26,9 | G | 37,6 | | | C | | |
| | | S=13.2 | T | 45,6 | T | 21,3 | T | 26,7 | | | 22,2 | | |
| 4 | GGG | 30,6 | A | 33,1 | A | 22,6 | A | 10,2 | K | N | R | R | S |
| (74) | AGG | 15,0 | C | 2,6 | C | 1,3 | C | 1,5 | 93,2 | 82,4 | 17,6 | 26,0 | 83,6 |
| | AAG | 7,6 | G | 56,3 | G | 66,2 | G | 77,7 | | | Y | K | |
| | | S=53.2 | T | 8,1 | T | 9,9 | T | 10,6 | | | 16,2 | 19,2 | |
| 5 | GAG | 12,0 | A | 30,0 | A | 71,6 | A | 20,6 | K | N | R | Q | S |
| (115) | GAT | 11,8 | C | 5,1 | C | 5,1 | C | 15,5 | 98,3 | 64,4 | 23,5 | 94,7 | 66,1 |
| | GAA | 8,8 | G | 58,5 | G | 18,4 | G | 35,6 | | | H | | |
| | | S=32.6 | T | 6,4 | T | 5,0 | T | 28,2 | | | 20,9 | | |
| | | | | | | | | | | | Y | | |
| | | | | | | | | | | | 19,1 | | |
| 6 | AAG | 9,1 | A | 40,6 | A | 59,9 | A | 15,7 | K | N | P | Q | S |
| (110) | TAG | 8,7 | C | 9,6 | C | 11,0 | C | 12,2 | 100 | 87,3 | 22 | 68,8 | 61,5 |
| | GAG | 8,0 | G | 23,6 | G | 15,7 | G | 49,0 | | | | | |
| | | S=25.8 | T | 26,2 | T | 13,4 | T | 23,2 | | | | | |
| 7 | AAT | 23,7 | A | 74,9 | A | 85,2 | A | 25,0 | K | N | Y | Q | S |
| (106) | AAA | 16,8 | C | 17,2 | C | 2,5 | C | 11,3 | 86,8 | 41,5 | 92,5 | 95,2 | 70,5 |
| | AAG | 16,6 | G | 5,9 | G | 11,5 | G | 30,0 | | T | | | |
| | | S=57.1 | T | 2,0 | T | 0,8 | T | 33,7 | | 24,5 | | | |
| 8 | GGG | 14,0 | A | 35,5 | A | 41,2 | A | 12,6 | K | N | Y | Q | S |
| (384) | TAG | 10,2 | C | 12,4 | C | 9,1 | C | 12,2 | 89,8 | 63,3 | 45,8 | 43,4 | 62,9 |
| | AAT | 6,9 | G | 30,7 | G | 37,3 | G | 52,4 | | | | | |
| | | S=31.1 | T | 21,4 | T | 12,4 | T | 22,8 | | | | | |
| 9 | TAG | 17,6 | A | 21,1 | A | 62,3 | A | 12,0 | K | N | C | Q | S |
| (134) | TAT | 9,9 | C | 13,0 | C | 2,2 | C | 13,9 | 92,5 | 76,1 | 17,9 | 74,6 | 72,7 |
| | AAG | 7,5 | G | 9,6 | G | 22,1 | G | 51,3 | | | S | | |
| | | S=35.0 | T | 56,3 | T | 13,4 | T | 22,7 | | | 16,4 | | |
| 10 | AAG | 20,2 | A | 64,3 | A | 78,9 | A | 18,1 | K | N | Y | Q | S |
| (399) | AAT | 14,7 | C | 5,4 | C | 6,7 | C | 10,6 | 96,0 | 59,5 | 53,6 | 69,2 | 70,1 |
| | AAA | 10,7 | G | 25,2 | G | 10,4 | G | 44,8 | | | | | |
| | | S=45.6 | T | 5,1 | T | 4,0 | T | 26,5 | | | | | |

| | | | | | | | | | | | | | |
|---|---|---|---|---|---|---|---|---|---|---|---|---|---|
| ¹Target and base frequencies correspond to cumulated intensity of cleavage as described in Materials and Methods). ² In each position, residues present in more than 15% of the cluster are indicated | | | | | | | | | | | | | |

Analysis of the residues found in each cluster showed strong biases for all randomized positions. None of the residues is mutated in all libraries used in this study, and the residues found in the I-CreI scaffold were expected to be overrepresented. Indeed, K28, N30 and S40 were the most frequent residues in all 10 clusters, and no conclusion for DNA/protein interactions can really be infered. However, Y33 was the most represented residue only in clusters 7, 8 and 10, whereas strong occurrence of other residues, such as H, R, G, T, C, P or S, was observed in the seven other clusters. The wild type Q38 residue was overrepresented in all clusters but one, R and K being more frequent in cluster 4.

Meanwhile, strong correlations were observed between the nature of residues 33 and 38 and substrate discrimination at positions ±10 and ±9 of the target.

Prevalence of Y33 was associated with high frequencies of adenine (74.9% and 64.3% in clusters 7 and 10, respectively), and this correlation was also observed, although to a lesser extent in clusters 4, 5 and 8. H33 or R33 were correlated with a guanine (63.0 %, 56.3 % and 58.5 %, in clusters 1, 4 and 5, respectively) and T33, C33 or S33 with a thymine (45.6 % and 56.3 % in clusters 3 and 9, respectively). G33 was relatively frequent in cluster 2, the cluster with the most even base representation in ±10. These results are consistent with the observations of Seligman and collaborators (Nucleic Acids Res., 2002, 30, 3870-3879), who showed previously that a Y33R or Y33H mutation shifted the specificity of I-CreI toward a guanine and Y33C, Y33T, Y33S (and also Y33L) towards a thymine in position ±10.

In addition, R38 and K38 were associated with an exceptional high frequency of guanine in cluster 4, while in all the other clusters, the wild type Q38 residue was overrepresented, as well as an adenine in ±9 of the target.

The structure of I-*Cre*I bound to its target (Chevalier et *al.,* 2003, precited; Jurica et al., 1998, precited) has shown that Y33 and Q38 contact two adenines in -10 and -9 (Figure 6C), and the results suggest that these interactions are probably maintained in many of the mutants. Similar results have been described previously for residue 44 and position ±4 (Arnould *et al.,* precited). However, comparing the results obtained for the 33/±10, 38/±9 and 44/±4 couples, shows that a given base can be correlated with different amino acid residues, depending on the position. For a guanine, the residues found mostly are R and H in position 33, R or K in 38, and K in 44, for adenine, Y in 33 and Q in 38 and 44, and for thymine, S, C or T in 33 and A in 44. In the three cases, no clear pattern is observed for cytosine. Thus, there is no universal "code", but rather a series of solutions for contacting each base, the best solution depending on a more general context, very similar to what has been observed with Zinc Finger proteins (Pabo *et al.,* precited).

## Claims

1. A I-*Cre*I meganuclease variant having new substrate specificity towards at least one of the nucleotides in positions ±8 and/or ±9 of a mutant I-*Cre*I target site wherein said variant comprises at least one mutation in one amino acid residue in positions 28, 30, 38 and 40.

2. The I-*Cre*I meganuclease variant according to claim 1 wherein said variant comprises at least one further mutation in at least one of positions 24, 26, 32, 33, 44, 68, 70, 75, 77, 80, 82, 85, 86, 87, 94, 96, 100, 103, 114, 115, 117, 125, 129, 131, 132, 140, 147, 151, 153, 154, 155, 157, 159 and 160, preferably in one of positions 44, 68 or 70.

3. The I-*Cre*I meganuclease variant according to anyone of claims 1 to 2 having a modified cleavage specificity for an I-*Cre*I DNA target sequence in which at least one nucleotide in either the ±8 to 10 triplets is altered.

4. The I-*Cre*I meganuclease variant according to anyone of claims 1 to 3 having at least the mutations selected in the group consisting of: Q28/N30/Y33/K38/R40, R28/N30/K33/R38/Q40, Q28/N30/R33/R38/R40, Q28/N30/Y33/K38/K40, Q28/N30/T33/Q38/K40, Q28/N30/R33/R38/K40, K28/N30/T33/Q38/R40, S28/N30/R33/S38/R40, N28/N30/Y33/Q38/R40, K28/N30/T33/R38/Q40, K28/N30/S33/R38/E40, Q28/N30/N33/Q38/K40, S28/N30/Y33/R38/K40, K28/N30/S33/R38/D40, K28/N30/R33/E38/R40, K28/N30/S33/R38/S40, R28/N30/R33/D38/R40, A28/N30/S33/Q38/R40, Q28/N30/Y33/R38/K40, Q28/N30/K33/R38/T40, R28/N30/A33/Y38/Q40, K28/N30/R33/Q38/E40, N28/N30/S33/R38/K40, N28/N30/S33/R38/R40, Q28/N30/Y33/Q38/K40, Q28/N30/Y33/Q38/R40, S28/N30/R33/Q38/R40, Q28/N30/R33/Q38/K40, E28/N30/R33/R38/K40, K28/N30/N33/Q38/A40, S28/N30/Y33/Q38/K40, T28/N30/R33/Q38/R40, Q28/N30/T33/Q38/R40, K28/N30/R33/T38/Q40, K28/N30/R33/T38/R40, Q28/N30/E33/D38/H40, R28/N30/Y33/N38/A40, Q28/N30/Y33/T38/R40, R28/N30/T33/R38/A40, H28/N30/Y33/D38/S40, Q28/N30/Y33/R38/A40, Q28/N30/Y33/A38/R40, S28/N30/Q33/A38/A40, Q28/N30/Y33/E38/K40, T28/N30/N33/Q38/R40, Q28/N30/Y33/R38/S40, K28/N30/R33/Q38/R40, Q28/N30/R33/A38/R40, Q28/N30/N33/Q38/R40, R28/N30/R33/E38/R40, K28/N30/R33/A38/R40, K28/N30/T33/A38/A40, K28/N30/R33/K38/A40, R28/N30/A33/K38/540, K28/N30/R33/N38/A40, T28/N30/E33/S38/D40, R28/N30/N33/Q38/D40, R28/N30/R33/Y38/Q40, K28/N30/Y33/Q38/N40, K28/N30/R33/S38/S40, K28/N30/R33/Y38/A40, A28/N30/N33/R38/K40, K28/N30/R33/A38/T40, K28/N30/R33/N38/Q40, T28/N30/T33/Q38/R40, K28/N30/R33/Q38/Y40, Q28/N30/S33/R38/K40, R28/N30/Y33/Q38/S40, Q28/N30/R33/Q38/R40, K28/N30/R33/A38/Q40, A28/N30/R33/Q38/R40, K28/N30/R33/Q38/Q40, K28/N30/R33/Q38/A40, K28/N30/T33/A38/S40, K28/A30/H33/R38/S40, K28/H30/H33/R38/S40, K28/D30/N33/H38/S40, K28/E30/S33/R38/S40, K28/H30/T33/P38/S40, K28/G30/H33/Y38/S40, K28/A30/R33/Q38/S40, K28/S30/R33/G38/S40, K28/S30/H33/H38/S40, K28/N30/H33/R38/S40, K28/R30/R33/E38/S40, K28/D30/G33/H38/S40, K28/R30/H33/G38/S40, K28/A30/N33/Q38/S40, K28/D30/H33/K38/S40, K28/K30/H33/R38/S40, K28/Q30/N33/Q38/S40, K28/Q30/T33/Q38/S40, K28/G30/R33/Q38/S40 K28/R30/P33/G38/S40, K28/R30/G33/N38/S40, K28/N30/A33/Q38/S40, K28/N30/H33/N38/S40, K28/H30/H33/A38/S40, K28/R30/G33/S38/S40, K28/S30/R33/Q38/S40, K28/T30/D33/H38/S40, K28/H30/H33/Q38/S40, K28/A30/D33/H38/S40, K28/S30/H33/R38/S40, K28/N30/R33/A38/S40, K28/S30/H33/Q38/S40, K28/D30/A33/H38/S40, K28/N30/H33/E38/S40, K28/D30/R33/T38/S40, K28/D30/R33/S38/S40, K28/A30/H33/Q38/S40, K28/R30/G33/T38/S40, K28/N30/H33/S38/S40, K28/Q30/H33/Q38/S40, K28/N30/H33/G38/S40, K28/N30/N33/Q38/S40, K28/N30/D33/Q38/S40, K28/D30/R33/G38/S40, K28/N30/H33/A38/S40, K28/H30/M33/A38/S40, K28/S30/S33/H38/S40, K28/G30/V33/A38/S40, K28/S30/V33/Q38/S40, K28/D30/V33/H38/S40, R28/D30/V33/Q38/S40, K28/G30/V33/Q38/S40, K28/G30/V33/T38/S340, K28/G30/V33/H38/S40, K28/G30/V33/R38/S40, K28/G30/V33/G38/S40, R28/A30/V33/G38/S40, R28/D30/V33/R38/S40, R28/N30/V33/Q38/S40,and N28/T30/V33/D38/S40.

5. The I-*Cre*I meganuclease variant according to anyone of claims 1 to 3 **characterized in that** said variant is able to cleave a DNA target sequence consisting of a mutant I-*Cre*I site wherein at least the "a" in position -8 and/or the "t" in position +8 has been replaced with a different nucleotide, said variant having at least the mutations selected in the group consisting of: Q28/N30/Y33/K38/R40, R28/N30/K33/R38/Q40, Q28/N30/R33/R38/R40, Q28/N30/Y33/K38/K40, Q28/N30/T33/Q38/K40, Q28/N30/R33/R38/K40, K28/N30/T33/Q38/R40, S28/N30/R33/S38/R40, N28/N30/Y33/Q38/R40, K28/N30/S33/R38/E40, Q28/N30/N33/Q38/K40, S28/N30/Y33/R38/K40, K28/N30/S33/R38/D40, K28/N30/R33/E38/R40, K28/N30/S33/R38/S40, R28/N30/R33/D38/R40, A28/N30/S33/Q38/R40, Q28/N30/Y33/R38/K40, Q28/N30/K33/R38/T40, R28/N30/A33/Y38/Q40, K28/N30/R33/Q38/E40, N28/N30/S33/R38/K40, N28/N30/S33/R38/R40, Q28/N30/Y33/Q38/K40, Q28/N30/Y33/Q38/R40, S28/N30/R33/Q38/R40, Q28/N30/R33/Q38/K40, E28/N30/R33/R38/K40, K28/N30/N33/Q38/A40, S28/N30/Y33/Q38/K40, T28/N30/R33/Q38/R40, Q28/N30/T33/Q38/R40, K28/N30/R33/T38/R40, Q28/N30/E33/D38/H40, R28/N30/Y33/N38/A40, Q28/N30/Y33/T38/R40, R28/N30/T33/R38/A40, H28/N30/Y33/D38/S40, Q28/N30/Y33/R38/A40, Q28/N30/Y33/A38/R40, S28/N30/Q33/A38/A40, Q28/N30/Y33/E38/K40, T28/N30/N33/Q38/R40, Q28/N30/Y33/R38/S40, K28/N30/R33/Q38/R40, Q28/N30/R33/A38/R40, Q28/N30/N33/Q38/R40, R28/N30/R33/E38/R40, K28/N30/R33/A38/R40, K28/N30/T33/A38/A40, K28/N30/R33/K38/A40, R28/N30/A33/K38/S40, K28/N30/R33/N38/A40, T28/N30/E33/S38/D40, R28/N30/N33/Q38/D40, R28/N30/R33/Y38/Q40, K28/N30/Y33/Q38/N40, K28/N30/R33/S38/S40, K28/N30/R33/Y38/A40, A28/N30/N33/R38/K40, K28/N30/R33/A38/T40, T28/N30/T33/Q38/R40, K28/N30/R33/Q38/Y40, Q28/N30/S33/R38/K40, R28/N30/Y33/Q38/S40, Q28/N30/R33/Q38/R40, A28/N30/R33/Q38/R40, K28/N30/R33/Q38/Q40, K28/N30/R33/Q38/A40, K28/N30/T33/A38/S40, K28/A30/H33/R38/S40, K28/H30/H33/R38/S40, K28/D30/N33/H38/S40, K28/E30/S33/R38/S40, K28/H30/T33/P38/S40, K28/G30/H33/Y38/S40, K28/A30/R33/Q38/S40, K28/S30/R33/G38/S40, K28/S30/H33/H38/S40, K28/N30/H33/R38/S40, K28/R30/R33/E38/S40, K28/D30/G33/H38/S40, K28/R30/H33/G38/S40, K28/A30/N33/Q38/S40, K28/D30/H33/K38/S40, K28/K30/H33/R38/S40, K28/Q30/N33/Q38/S40, K28/Q30/T33/Q38/S40, K28/G30/R33/Q38/S40 K28/R30/P33/G38/S40, K28/R30/G33/N38/S40, K28/N30/A33/Q38/S40, K28/N30/H33/N38/S40, K28/H30/H33/A38/S40, K28/R30/G33/S38/S40, K28/S30/R33/Q38/S40, K28/T30/D33/H38/S40, K28/H30/H33/Q38/S40, K28/A30/D33/H38/S40, K28/S30/H33/R38/S40, K28/N30/R33/A38/S40, K28/S30/H33/Q38/S40, K28/D30/A33/H38/S40, K28/N30/H33/E38/S40, K28/D30/R33/T38/S40, K28/D30/R33/S38/S40, K28/A30/H33/Q38/S40, K28/R30/G33/T38/S40, K28/N30/H33/S38/S40, K28/Q30/H33/Q38/S40, K28/N30/H33/G38/S40, K28/N30/N33/Q38/S40, K28/N30/D33/Q38/S40, K28/D30/R33/G38/S40, K28/N30/H33/A38/S40, K28/H30/M33/A38/540, K28/S30/S33/H38/S40, K28/G30/V33/A38/S40, K28/S30/V33/Q38/S40, K28/D30/V33/H38/S40, R28/D30/V33/Q38/S40, K28/G30/V33/Q38/S40 K28/G30/V33/T38/S40, K28/G30/V33/H38/S40, K28/G30/V33/R38/S40, K28/G30/V33/G38/S40, R28/A30/V33/G38/S40, R28/D30/V33/R38/S40, R28/N30/V33/Q38/S40, and N28/T30/V33/D38/S40.

6. The I-*Cre*I meganuclease variant according to anyone of claims 1 to 3 **characterized in that** said variant is able to cleave a DNA target sequence consisting of a mutant I-*Cre*I site wherein at least the "a" in position -9 and/or the "t" in position +9 has been replaced with a different nucleotide, said variant having at least the mutations selected in the group consisting of: Q28/N30/Y33/K38/R40, R28/N30/K33/R38/Q40, Q28/N30/R33/R38/R40, Q28/N30/Y33/K38/K40, Q28/N30/T33/Q38/K40, Q28/N30/R33/R38/K40, K28/N30/T33/Q38/R40, S28/N30/R33/S38/R40, K28/N30/T33/R38/Q40, K28/N30/S33/R38/E40, S28/N30/Y33/R38/K40, K28/N30/S33/R38/D40, K28/N30/R33/E38/R40, K28/N30/S33/R38/S40, R28/N30/R33/D38/R40, Q28/N30/Y33/R38/K40, R28/N30/A33/Y38/Q40, N28/N30/S33/R38/K40, N28/N30/S33/R38/R40, E28/N30/R33/R38/K40, K28/N30/N33/Q38/A40, K28/N30/R33/T38/Q40, K28/N30/R33/T38/R40, Q28/N30/E33/D38/H40, R28/N30/T33/R38/A40, H28/N30/Y33/D38/S40, K28/N30/R33/Q38/R40, Q28/N30/R33/A38/R40, R28/N30/R33/E38/R40, K28/N30/R33/A38/R40, K28/N30/T33/A38/A40, K28/N30/R33/K38/A40, K28/N30/R33/N38/A40, R28/N30/R33/Y38/Q40, K28/N30/R33/S38/S40, A28/N30/M33/R38/K40, K28/N30/R33/A38/T40, K28/N30/R33/N38/Q40, T28/N30/T33/Q38/R40, K28/N30/R33/Q38/Y40, Q28/N30/S33/R38/K40, K28/N30/R33/A38/Q40, A28/N30/R33/Q38/R40, K28/N30/R33/Q38/A40, K28/N30/T33/A38/S40, K28/A30/H33/R38/S40, K28/H30/H33/R38/S40, K28/D30/N33/H38/S40, K28/E30/S33/R38/S40, K28/S30/R33/G38/S40, K28/S30/H33/H38/S40, K28/N30/H33/R38/S40, K28/R30/R33/E38/S40, K28/D30/G33/H38/S40, K28/D30/H33/K38/S40, K28/K30/H33/R38/S40, K28/Q30/N33/Q38/S40, K28/R30/G33/N38/S40, K28/N30/H33/N38/S40, K28/H30/H33/A38/S40, K28/R30/G33/S38/S40, K28/T30/D33/H38/S40, K28/A30/D33/H38/S40, K28/S30/H33/R38/S40, K28/N30/R33/A38/540, K28/D30/A33/H38/S40, K28/D30/R33/T38/S40, K28/D30/R33/S38/S40, K28/R30/G33/T38/S40, K28/N30/H33/S38/S40, K28/N30/H33/G38/S40, K28/N30/N33/Q38/S40, K28/D30/R33/G38/S40, K28/N30/H33/A38/S40, K28/H30/M33/A38/S40, K28/S30/S33/H38/S40, K28/G30/V33/A38/S40, K28/D30/V33/H38/S40, K28/G30/V33/T38/S340, K28/G30/V33/H38/S40, K28/G30/V33/R38/S40, and K28/G30/V33/G38/S40.

7. The I-*Cre*I meganuclease variant according to anyone of claims 1 to 6 **characterized in that** said I-*Cre*I meganuclease variant is an homodimer.

8. The I-*Cre*I meganuclease variant according to anyone of claims 1 to 6 **characterized in that** said I-*Cre*I meganuclease variant is an heterodimer.

9. The I-*Cre*I meganuclease variant according to anyone of claims 1 to 6 **characterized in that** said I-*Cre*I meganuclease variant is a single-chain chimeric endonuclease.

10. The I-*Cre*I meganuclease variant according to claim 8 **characterized in that** said heterodimer results from the fusion of two I-*Cre*I monomers.

11. The I-*Cre*I meganuclease variant according to any one of preceding claims for the preparation of a medicament for preventing, improving or curing a disease caused by an infectious agent that presents a DNA intermediate, in an individual in need thereof, said medicament being administrated by any means to said individual.
